# EUROPEAN PATENT SPECIFICATION

(11) **EP 4 504 112 B1**
(45) Date of publication and mention of the grant of the patent: **05.08.2026**
(21) Application number: 23719621.7
(22) Date of filing: 03.04.2023
(51) Int. Cl.: A61F 13/49, A61F 13/494, A61F 13/15

(54) **ABSORBENT ARTICLES INCLUDING A WAIST PANEL**
SAUGFÄHIGE ARTIKEL MIT EINER TAILLENTAFEL
ARTICLES ABSORBANTS COMPRENANT UN PANNEAU DE TAILLE

(30) Priority: 04.04.2022 US 202263327026 P
(43) Date of publication of application: 12.02.2025
(73) Proprietor: The Procter & Gamble Company, Cincinnati, OH 45202 (US)
(72) Inventor: WILLIAMS, Tanner, Laurie, Cincinnati, Ohio 45202 (US); WILLHAUS, Keith, Richard, Cincinnati, Ohio 45202 (US); NAYLOR, Jason, Edward, Cincinnati, Ohio 45202 (US); MAUTHNER, Erin, A., Cincinnati, Ohio 45202 (US); RINNERT, Thorsten, 65824 Schwalbach Am Taunus (DE)
(74) Representative: P&G Patent Germany
(86) International application number: PCT/US2023/065262
(87) International publication number: WO 2023/196768

(56) References cited:
- US-A1- 2020 375 815
- US-A1- 2021 346 211

## Description

### FIELD OF THE INVENTION

The present disclosure relates to absorbent articles including waist panels, and more particularly, to waist panels forming a pocket and having bonding arrangements that define reinforced zones.

### BACKGROUND OF THE INVENTION

Along an assembly line, various types of articles, such as for example, diapers and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from an advancing web or webs are combined with other individual components created from other advancing webs. Webs of material and component parts used to manufacture diapers may include: backsheets, topsheets, leg cuffs, waistbands, absorbent core components, front and/or back ears, fastening components, and various types of elastic webs and parts such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles.

Some absorbent articles, such as diapers, have components that include waist panels, which may also be referred to as waistbands. In some configurations, waistbands may be provided as a single layer of elastic material, such as an elastic film. In some configurations, the waistbands may be provided as an elastic laminate that may include elastic material bonded to one or more substrates such as nonwovens, wherein the elastic material may include an elastic film and/or elastic strands. In some assembly operations, the waistbands are joined to an advancing carrier web, such as a continuous topsheet or backsheet web, while the waistbands are in a stretched condition. As such, when the waistbands relax, the carrier web gathers to form corrugations. The resulting laminate is stretchable to the extent that the corrugations allow the waistband to elongate.

When manufacturing diapers, the waistband may be provided as a continuous length of waistband material that may be stretched; cut into discrete waistbands; and bonded with the advancing carrier web, such as a continuous topsheet or backsheet web, while the waistband is in a stretched state. With some diapers, it may be desirable to include a front waistband in a front waist region and a back waistband in an opposing back waist region. Some assembly operations may apply a piece of waistband material to the advancing carrier web that is subsequently cut into separate front and back waistbands when the advancing carrier web is subject to the final knife cut that separates the carrier web into discrete diapers. In turn, the front and back waistbands may be created from the same continuous length of waistband material.

In some configurations, it may be desirable to provide diapers with front and/or rear waistbands having regions that are bonded with a topsheet, and one or more regions that are not bonded (unattached) with the topsheet, thereby forming a pocket between the waistband and the topsheet. Such a pocket may help contain bodily exudates and/or help prevent leaks from an absorbent article. In some configurations, the pocket may be formed by attaching a waistband to a topsheet along three edges of the waistband wherein a fourth edge of the waistband remains unattached to the topsheet.

There are sometimes challenges associated with manufacturing absorbent articles with waistbands having pockets. For example, the intersections between bonded regions along the three edges of the waistband may leave small gaps between the waistband and the topsheet. Thus, such gaps may be susceptible to undesirable leakage during use. In addition, depending on a particular manufacturing configuration, the unattached edge of a waistband may be oriented so as define a leading edge of the waistband while advancing through various assembly operations. As such, in some instances, air may be forced into the pocket while the waistband is advancing through assembly operations, causing the waistband to act like a sail or parachute by repeatedly lifting and/or separating from the topsheet and/or otherwise deforming the waistband shape. Such repeated lifting and/or deformation may cause the waistband to be partially or whole torn away from the topsheet. In some configurations, frictional forces acting on diaper components advancing along various assembly apparatuses, such as folding plows, may drag or pull on the unattached edge, in turn, causing removal of a portion or an entirety of the waistband.

Consequently, it would be beneficial to provide absorbent articles with waist panels configured with an edge that is unattached from other diaper components, such as a topsheet, so as to form a pocket during use, and wherein bonded regions between the waistband and an absorbent article component, such as a topsheet, may have reinforced regions so as to help prevent and/or reduce instances of separation of the waistband from such absorbent article components during assembly and/or use as well as reduce the likelihood of leakage during use.

### SUMMARY OF THE INVENTION

In one form, an absorbent article comprises: a front waist region, a back waist region, and a crotch region disposed between the front and back waist regions; a chassis comprising a topsheet, a backsheet, and an absorbent core positioned between the topsheet and the backsheet; a first leg gasketing element and a second leg gasketing element connected with the chassis and extending from the front waist region to the back waist region; a first waist panel positioned in the front waist region or the back waist region, the first waist panel comprising an inboard lateral edge, an outboard lateral edge, a first longitudinal edge, and a second longitudinal edge, wherein the first waist panel further comprises a first lateral end region adjacent the first longitudinal edge and a second lateral end region adjacent the second longitudinal edge, wherein the first and second lateral end regions are separated by a central region; a first zone of mechanical bonds in the first lateral end region, the first zone of mechanical bonds bonding the first waist panel with at least one of the chassis and the first leg gasketing element; a second zone of mechanical bonds in the second lateral end region, the second zone of mechanical bonds bonding the first waist panel with at least one of the chassis and the second leg gasketing element; a first zone of adhesive in the first lateral end region adjacent the inboard lateral edge and bonding the first waist panel with at least one of the chassis and the first leg gasketing element; a second zone of adhesive in the second lateral end region adjacent the inboard lateral edge and bonding the first waist panel with at least one of the chassis and the second leg gasketing element; and wherein the first waist panel is unattached to the chassis between the first and second lateral end regions.

In another form, an absorbent article comprises: a front waist region, a back waist region, and a crotch region disposed between the front and back waist regions; a chassis comprising a topsheet, a backsheet, and an absorbent core positioned between the topsheet and the backsheet; a first leg gasketing element and a second leg gasketing element connected with the chassis and extending from the front waist region to the back waist region; a zone of adhesive adjacent the outboard lateral edge and extending laterally through the central region, the zone of adhesive connecting the first waist panel with the chassis; a first waist panel positioned in the front waist region or the back waist region, the first waist panel comprising an inboard lateral edge, an outboard lateral edge, a first longitudinal edge, and a second longitudinal edge, wherein the first waist panel further comprises a first lateral end region adjacent the first longitudinal edge and a second lateral end region adjacent the second longitudinal edge, wherein the first and second lateral end regions are separated by a central region; a first zone of mechanical bonds in the first lateral end region, the first zone of mechanical bonds bonding the first waist panel with at least one of the chassis and the first leg gasketing element; a second zone of mechanical bonds in the second lateral end region, the second zone of mechanical bonds bonding the first waist panel with at least one of the chassis and the second leg gasketing element; wherein the first waist panel is unattached to the chassis between the first and second lateral end regions and between the inboard lateral edge and the zone of adhesive; and wherein a portion of the first zone of mechanical bonds and a portion of the second zone of mechanical bonds overlap with portions of the zone of adhesive.

In yet another form, a method for assembling absorbent articles comprises steps of: advancing a carrier substrate in a machine direction; providing an elastic part comprising a first longitudinal edge and a second longitudinal edge separated from the first longitudinal edge in a cross direction, the elastic part further comprising a first edge and a second edge separated from the first edge in machine direction; the elastic part further comprising a first end region and a second end region separated from the first end region in the cross direction by a central region; adhesively bonding each elastic part with the carrier substrate with a first zone of adhesive in the first end region adjacent the first edge; adhesively bonding each elastic part with the carrier substrate with a second zone of adhesive in the second end region adjacent the first edge; mechanically bonding the first end region of each elastic part to the carrier substrate with a first zone of mechanical bonds in the first end region and extending between the first edge and the second edge; mechanically bonding the second end region of each elastic part to the carrier substrate with a second zone of mechanical bonds in the second end region and extending between the first edge and the second edge, and wherein the elastic part is unattached to the carrier substrate between the first and second end regions.

In still another form, a method for assembling absorbent articles comprises steps of: advancing a carrier substrate in a machine direction; providing an elastic part comprising a first longitudinal edge and a second longitudinal edge separated from the first longitudinal edge in a cross direction, the elastic part further comprising a first edge and a second edge separated from the first edge in machine direction; the elastic part further comprising a first end region and a second end region separated from the first end region in the cross direction by a central region; adhesively bonding each elastic part with the carrier substrate with a zone of adhesive adjacent the second edge and extending through the central region; mechanically bonding the first end region of each elastic part to the carrier substrate with a first zone of mechanical bonds in the first end region and extending between the first edge and the second edge; mechanically bonding the second end region of each elastic part to the carrier substrate with a second zone of mechanical bonds in the second end region and extending between the first edge and the second edge, wherein the elastic part is unattached to the carrier substrate between the first and second end regions and between the first edge and the zone of adhesive; and wherein a portion of the first zone of mechanical bonds and a portion of the second zone of mechanical bonds overlap with portions of the zone of adhesive.

### BRIEF DESCRIPTION OF THE DRAWINGS

Figure 1A is a partially cut away plan view of an absorbent article in the form of a taped diaper that may include one or more substrates assembled in accordance with the present disclosure with the portion of the diaper that faces away from a wearer oriented towards the viewer.
Figure 1B is a plan view of the absorbent article of Figure 1A that may include one or more substrates assembled in accordance with the present disclosure with the portion of the diaper that faces toward a wearer oriented towards the viewer.
Figure 2 is a detailed view of a first waist panel with the portion of the diaper that faces toward a wearer oriented towards the viewer.
Figure 2A is a detailed view of the first waist panel from Figure 2 illustrating bonding configurations.
Figure 2B is a detailed view of the first waist panel from Figure 2A showing a first bond configuration.
Figure 2C is a detailed view of the first waist panel from Figure 2A showing a second bond configuration.
Figure 3 is a detailed view of a second waist panel with the portion of the diaper that faces toward a wearer oriented towards the viewer.
Figure 3A is a detailed view of a second waist panel from Figure 3 illustrating bonding configurations.
Figure 3B is a detailed view of the second waist panel from Figure 3A showing a first bond configuration.
Figure 3C is a detailed view of the first waist panel from Figure 3A showing a second bond configuration.
Figure 4 is a schematic side view of an apparatus for bonding elastic parts to an advancing carrier web.
Figure 4A is a detailed schematic view of a bonding apparatus with a pressing surface comprising an ultrasonic bonding device.
Figure 5 is a view of a carrier substrate taken along section 5-5 in Figure 4.
Figure 5A is a view of a carrier substrate with leg cuffs taken along section 5-5 in Figure 4.
Figure 6 is a view of a continuous elastic substrate taken along section 6-6 in Figure 4.
Figure 7 is a view of a continuous elastic substrate with discrete patches of adhesive taken along section 7-7 in Figure 4.
Figure 8 is a view of a discrete elastic part laid out flat with a zone of adhesive thereon taken along section 8-8 in Figure 4.
Figure 9 is a view of a cutting device, transfer device, and bonding device taken along section 9-9 in Figure 4.
Figure 10 is a view of the transfer device and bonding device taken along section 10-10 in Figure 9.
Figure 11 is a detailed view of the spreader mechanism taken along section 11-11 in Figure 10.
Figure 11A is a detailed view of radially protruding nubs on an outer rim of a disk.
Figure 12 is a view of a stretched discrete elastic part laid out flat with a zone of adhesive thereon taken along section 12-12 in Figure 4.
Figure 13 is a detailed cross sectional view of a pattern roll from Figure 9 showing bonding elements extending radially outward from an outer circumferential surface taken along line 13-13.
Figure 13A is a detailed view of a portion of the outer circumferential surface of the pattern roll showing bonding elements from Figure 13 taken along line 13A-13A.
Figure 14 is a view of a laminate including the elastic part and the carrier substrate taken along section 14-14 in Figure 4.
Figure 14A is a view of the laminate of Figure 14 including the elastic part and the carrier substrate after being subjected to a final knife cut operation that applies cut lines through the carrier substrate and discrete elastic parts.
Figure 14B is a view of another configuration of the laminate including the elastic part and the carrier substrate taken along section 14-14 in Figure 4.
Figure 14C is a view of another configuration of the laminate of Figure 4 including the elastic part and the carrier substrate after being subjected to a final knife cut operation that applies cut lines through the carrier substrate and discrete elastic parts.
Figure 15 is a view of the laminate including the elastic part and the carrier substrate taken along section 15-15 in Figure 14.
Figure 16 is a view of the carrier substrate and adhesive taken along section 16-16 in Figure 4.
Figure 17 is a cross sectional view of an example waist panel.
Figure 18 is a cross-sectional view of the waist panel from Figure 17 in a relaxed, contracted condition.

### DETAILED DESCRIPTION OF THE INVENTION

The following term explanations may be useful in understanding the present disclosure:
"Absorbent article" is used herein to refer to consumer products whose primary function is to absorb and retain soils and wastes. Absorbent articles can comprise sanitary napkins, tampons, panty liners, interlabial devices, wound dressings, wipes, disposable diapers including taped diapers and diaper pants, inserts for diapers with a reusable outer cover, adult incontinent diapers, adult incontinent pads, and adult incontinent pants. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

"Consolidation," "consolidating," and "consolidated" refers to a material undergoing a reduction in elongation from a first stretched length to a second stretched length that is less than the first stretched length and greater than zero.

"Relaxed state" defines a length of material when not stretched by an applied force.

In the context of the present description, an elongation of 0% refers to a material in relaxed state having a relaxed length of L, and elongation of 150% represents 2.5x the relaxed length, L, of the material. For example, an elastic film having a relaxed length of 100 millimeters would have a length of 250 millimeters at 150% elongation. And an elastic film having a relaxed length of 100 millimeters would have a length of 180 millimeters at 80% elongation.

In the context of the present description, a contraction of 60% represents 0.6x contraction of an initial stretch length, L, of a material. For example, an elastic film having an initial stretch length of 250 millimeters would have a contracted length of 100 millimeters at 60% contraction. And an elastic film having an initial stretch length of 180 millimeters would have a length of 100 millimeters at 44% contraction.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily twodimensional (i.e., in an XY plane) and whose thickness (in a Z direction) is relatively small (i.e., 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. In some configurations, a nonwoven may comprise a polyolefin based nonwoven, including but not limited to nonwovens having polypropylene fibers and/or polyethylene fibers and/or bicomponent fibers comprising a polyolefin. Nonlimiting examples of suitable fibers include spunbond, spunlaid, meltblown, spunmelt, solvent-spun, electrospun, carded, film fibrillated, melt-film fibrillated, air-laid, dry-laid, wet-laid staple fibers, and other nonwoven web materials formed in part or in whole of polymer fibers as known in the art, and workable combinations thereof. Nonwovens do not have a woven or knitted filament pattern. It is to be appreciated that nonwovens having various basis weights can be used in accordance with the methods herein. For example, some nonwovens may have a basis weight of at least about 8 gsm, 12 gsm, 16 gsm, 20 gsm, 25 gsm, 30 gsm, 40 gsm, or 65 gsm. Some nonwovens may have basis weight of about 8 gsm to about 65 gsm, specifically reciting all 1 gsm increments within the above-recited ranges and all ranges formed therein or thereby.

It is to be appreciated that films having various basis weights can be used in accordance with the methods herein. For example, some films may have a basis weight of at least about 8 gsm, 12 gsm, 16 gsm, 20 gsm, 25 gsm, 30 gsm, 40 gsm, or 60 gsm. Some films may have basis weight of about 5 gsm to about 150 gsm, specifically reciting all 1 gsm increments within the above-recited ranges and all ranges formed therein or thereby.

It is to be appreciated that elastic films discussed herein may comprise various materials and/or components. Some elastomeric compositions may comprise thermoplastic elastomers selected from the group consisting of Styrenic block copolymers, poly-esters, polyurethanes, polyether amides, and combinations thereof. Suitable styrenic block copolymers may be diblock, triblock, tetrablock, or other multi-block copolymers having at least one styrenic block. Exemplary styrenic block copolymers include styrene-butadiene-styrene, styrene-isoprene-styrene, styreneethylene/butylenes-styrene, styrene-ethylene/propylene-styrene, and the like. Commercially available styrenic block copolymers include KRATON (styrenic block copolymer; available from the Kraton Chemical Company, Houston, TX), SEPTON (styrenic block copolymer; available from Kuraray America, Inc., New York, NY), VECTOR (styrenic block copolymer; available from TSRC Dexco Chemical Company, Houston, TX) can be used. Additional commercially available elastomers include ESTANE (polyurethane; available from Lubrizol, Inc, Ohio), PEBAX (polyether block amide; available from Arkema Chemicals, Philadelphia, PA), and HYTREL (polyester; available from DuPont, Wilmington, DE).

Semi-crystalline, or metallocene polyolefins may be used in disposable absorbent products. The polyolefin elastomer materials herein may include, but are not limited to, any polymers or copolymers of polyolefins such as polyethylene and polypropylene. Examples of elastomeric polypropylenes include an elastic random poly(propylene/olefin) copolymer, an isotactic polypropylene containing stereo-irregularity, an isotactic/atactic polypropylene block copolymer, an isotactic polypropylene/random poly(propylene/olefin) copolymer block copolymer, a stereoblock elastomeric polypropylene, a syndiotactic polypropylene block poly(ethylene-co-propylene) block syndiotactic polypropylene triblock copolymer, an isotactic polypropylene block regioirregular polypropylene block isotactic polypropylene triblock copolymer, a polyethylene random (ethylene/olefin) copolymer block copolymer, a reactor blend polypropylene, a very low density polypropylene (or, equivalently, ultra low density polypropylene), a metallocene polypropylene, and blends or combinations thereof. Some homopolyolefins and random copolymers, as well as blends of such random copolymers, known by tradenames Vistamaxx ^{™} available from ExxonMobil and VERSIFY ^{™} from Dow, tend to show elastic performance. In some embodiments, two or more elastomers may be blended to achieve the desired elastic performance. For example, Styrenic block copolymer can be blended with polyolefin based elastomers, or polypropylene based elastomer can be blended with other polyolefin based elastomers.

Components of the absorbent articles described herein may at least partially be comprised of bio-based content as described in U.S. Pub. No. 2007/0219521 A1. For example, the superabsorbent polymer component may be bio-based via their derivation from bio-based acrylic acid. Bio-based acrylic acid and methods of production are further described in U.S. Pub. No. 2007/0219521 A1 and U.S. Patient Nos. 8,703,450; 9,630,901 and 9,822,197. Other components, for example nonwoven and film components, may comprise bio-based polyolefin materials. Bio-based polyolefins are further discussed in U.S. Pub. Nos. 2011/0139657 A1, 2011/0139658 A1, 2011/0152812 A1, and 2016/0206774 A1, and U.S. Patent No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pub. No. 2019/0192723 A1, published on June 27, 2019.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

Aspects of the present disclosure relate to absorbent articles with waist panels forming a pocket and having bonding arrangements that define reinforced zones. As discussed below, an absorbent article may comprise: a front waist region, a back waist region, and a crotch region disposed between the front and back waist regions. In addition, an absorbent article may comprise a chassis comprising a topsheet, a backsheet, and an absorbent core positioned between the topsheet and the backsheet. First and second leg gasketing elements may be connected with the chassis that extend from the front waist region to the back waist region. A waist panel may be connected with the chassis and positioned in the front waist region or the back waist region. In some configurations, similarly configured or differently configured waist panels may be positioned in both the front waist region and the back waist region. The waist panel may comprise an inboard lateral edge, an outboard lateral edge, a first longitudinal edge, and a second longitudinal edge, and wherein the first waist panel further comprises a first lateral end region adjacent the first longitudinal edge and a second lateral end region adjacent the second longitudinal edge, wherein the first and second lateral end regions are separated by a central region. The waist panel may be bonded with at least one of the chassis and the leg gasketing elements in first and second zones of mechanical bonds in the first and second lateral end regions. The waist panel may also be bonded with at least one of the chassis and the leg gasketing elements in first and second zones of adhesive in the first and second lateral end regions. The first and second zones of adhesive may be adjacent the inboard lateral edge and may overlap the first and second mechanical bond zones. In addition, a third zone of adhesive adjacent the outboard lateral edge of the waist panel may bond the waist panel with the chassis. The mechanical bond zones may also overlap with the third zone of adhesive. The waist panel may be unattached to the chassis between the first and second lateral end regions and between the inboard lateral edge and the third zone of adhesive to form a pocket. The placement of zones of adhesive adjacent the inboard lateral edge and/or the overlapping of zones of adhesive and zones of mechanical bonds may help to reinforce and/or strengthen bonded regions to help prevent and/or reduce instances of separation of the waist panel from other absorbent article components during assembly operations and/or during use. The overlapping of zones of adhesive and zones of mechanical bonds may also help to reduce the likelihood of undesirable leakage in overlapped areas during use. The waist panels are discussed below in the context of absorbent articles that may be configured as taped diapers or pant diapers.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Pub. Nos. 2013/0072887 A1; 2013/0211356 A1; and 2013/0306226 A1

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed). Example diaper pants in various configurations are disclosed in U.S. Patent Nos. 4,940,464; 5,092,861; 5,246,433; 5,569,234; 5,897,545; 5,957,908; 6,120,487; 6,120,489; 7,569,039 and U.S. Pub. Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1,

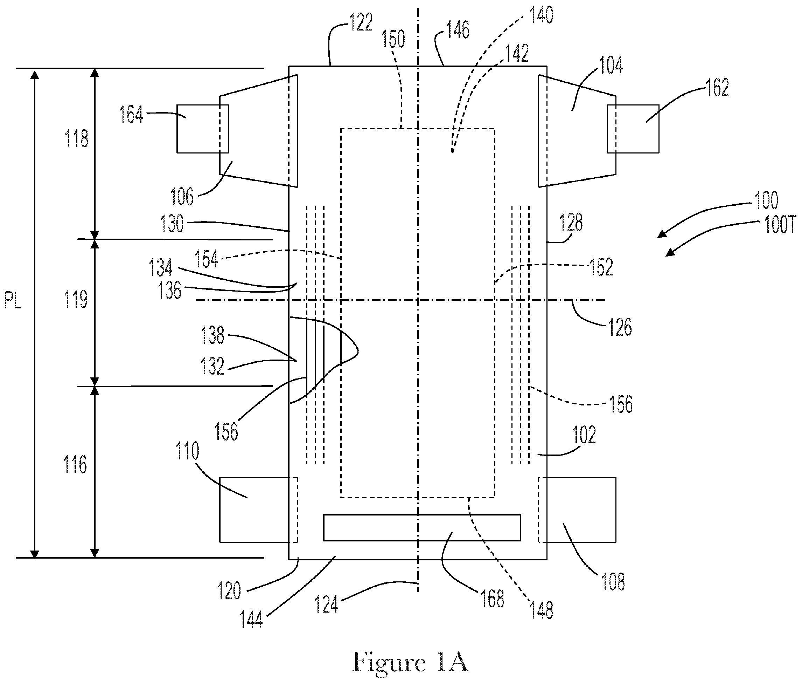

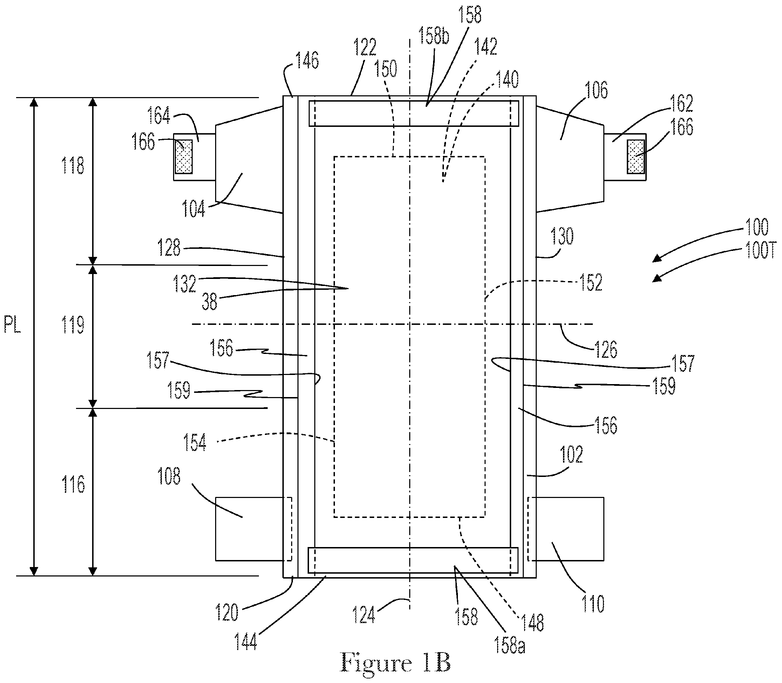

For the purposes of a specific illustration, Figures 1A and 1B show an example of an absorbent article 100 that may be assembled in accordance with the present disclosure. In particular, Figure 1A shows one example of a plan view of an absorbent article 100 configured as a taped diaper 100T, with the portion of the diaper that faces away from a wearer oriented towards the viewer. And Figure 1B shows a plan view of the diaper 100 with the portion of the diaper that faces toward a wearer oriented towards the viewer. The taped diaper 100T shown in Figures 1A and 1B includes an absorbent chassis 102, first and second rear side panels 104 and 106; and first and second front side panels 108 and 110.

As shown in Figures 1A and 1B, the absorbent article 100 and the chassis 102 each include a first waist region 116, a second waist region 118, and a crotch region 119 disposed intermediate the first and second waist regions. The first waist region 116 may be configured as a front waist region, and the second waist region 118 may be configured as a back waist region. In some embodiments, the length of each of the front waist region, back waist region, and crotch region may be 1/3 of the length of the absorbent article 100. The absorbent article 100 may also include a laterally extending first waist edge 120 in the first waist region 116, wherein the first waist edge 120 may be configured as a front waist edge. In addition, the absorbent article 100 may include a laterally extending second waist edge 122 in the second waist region 118, wherein the second waist edge 122 may be configured as a back waist edge. To provide a frame of reference for the present discussion, the diaper 100T in Figures 1A and 1B is shown with a longitudinal axis 124 and a lateral axis 126. The longitudinal axis 124 may extend through a midpoint of the front waist edge 120 and through a midpoint of the back waist edge 122. And the lateral axis 126 may extend through a midpoint of a first longitudinal or right side edge 128 and through a midpoint of a second longitudinal or left side edge 130.

As shown in Figures 1A and 1B, the absorbent article 100 includes an inner, wearer facing surface 132, and an outer, garment facing surface 134. As such, it is also to be appreciated that the various components of the absorbent article described below may each include inner, wearer facing surfaces 132, and an outer, garment facing surfaces 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the absorbent article 100 may also include other features, such as leg gasketing elements, waist panels, and/or flaps, e.g., side panels and/or ears, to enhance the fits around the legs and waist of the wearer, to enhance the fit around the legs of the wearer.

As shown in Figures 1A and 1B, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 1A, the laterally extending end edges 144 and 146 may form a portion of the laterally extending front waist edge 120 in the front waist region 116 and a portion of the longitudinally opposing and laterally extending back waist edge 122 in the back waist region 118. The distance between the first lateral end edge 144 and the second lateral end edge 146 may define a pitch length, PL, of the chassis 102. When the absorbent article 100 is worn on the lower torso of a wearer, the front waist edge 120 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the absorbent article 100 may also be made laterally extensible. The additional extensibility may help allow the absorbent article 100 to conform to the body of a wearer during movement by the wearer. The additional extensibility may also help, for example, the user of the absorbent article 100, including a chassis 102 having a particular size before extension, to extend the front waist region 116, the back waist region 118, or both waist regions of the absorbent article 100 and/or chassis 102 to provide additional body coverage for wearers of differing size, i.e., to tailor the absorbent article to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the absorbent article 100 may include a backsheet 136. The backsheet 136 may also define the outer surface 134 of the chassis 102. The backsheet 136 may be impervious to fluids (e.g., menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the absorbent article 100, such as bedsheets, pajamas and undergarments. The backsheet 136 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet 136 may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the absorbent article 100.

Also described above, the absorbent article 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539,

As mentioned above, the absorbent article 100 may also include an absorbent assembly 140 that is joined to the chassis 102. As shown in Figures 1A and 1B, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the absorbent article. Exemplary absorbent structures for use as the absorbent core of the present disclosure are described in U.S. Patent Nos. 4,610,678; 4,673,402; 4,888,231; and 4,834,735

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Pub. Nos. 2004/0158212 A1 and 2004/0097895 A1

In some configurations, the absorbent assembly 140 may include an acquisition system disposed between the topsheet 138 and a wearer facing side of the absorbent core 142. The acquisition system may be in direct contact with the absorbent core 142 and may comprise a single layer or multiple layers, such as an upper acquisition layer (also referred to herein as a first acquisition layer) facing towards the wearer's skin and a lower acquisition layer (also referred to herein as a second acquisition layer) facing the garment of the wearer. In some configurations, the acquisition system may function to receive a surge of liquid, such as a gush of urine. As such, the acquisition system may serve as a temporary reservoir for liquid until the absorbent core 142 can absorb the liquid. Exemplary acquisition systems and associated manufacturing processes are described in U.S. Patent Nos. 8,603,277 and 8,568,566; U.S. Pub. Nos. 2012/0316046 A1 and 2014/0163504 A1

In some configurations, the acquisition system may include chemically cross-linked cellulosic fibers. Such cross-linked cellulosic fibers may have various absorbency properties. Exemplary chemically cross-linked cellulosic fibers are disclosed in U.S. Patent No. 5,137,537. Citric acid is an exemplary cross-linking agent. In some embodiments, polyacrylic acids may be used. In some configurations, the cross-linked cellulosic fibers may be crimped, twisted, or curled, or a combination thereof including crimped, twisted, and curled.

In some configurations, one or both of the upper acquisition layer and lower acquisition layer may include a nonwoven, which may be hydrophilic. Further, according to some configurations, one or both of the upper acquisition layer and lower acquisition layer may comprise chemically cross-linked cellulosic fibers, which may or may not form part of a nonwoven material. In some embodiments, the upper acquisition layer may comprise a nonwoven, without the cross-linked cellulosic fibers, and the lower acquisition layer may comprise the chemically cross-linked cellulosic fibers. Further, in some configurations, the lower acquisition layer may comprise the chemically cross-linked cellulosic fibers mixed with other fibers such as natural or synthetic polymeric fibers. According to some embodiments, such other natural or synthetic polymeric fibers may include high surface area fibers, thermoplastic binding fibers, polyethylene fibers, polypropylene fibers, PET fibers, rayon fibers, lyocell fibers, and mixtures thereof.

Exemplary absorbent assemblies 140, absorbent cores 142, and associated components that may be adapted for use with the present disclosure are described in U.S. Patent Nos. 4,610,678; 4,673,402; 4,888,231; 4,834,735; 4,888,231; 5,260,345; 5,387,207; 5,397,316; 8,603,277; and 8,568,566; and U.S. Pub. Nos. 2012/0316046 A1 and 2014/0163504 A1

Taped diapers may be manufactured and provided to consumers in a configuration wherein the front waist region and the back waist region are not fastened, pre-fastened, or connected to each other as packaged, prior to being applied to the wearer. For example, the taped diaper 100T may be folded about a lateral centerline with the interior surface 132 of the first waist region 116 in surface to surface contact with the interior surface 132 of the second waist region 118 without fastening or joining the waist regions together. The rear side panels 104 and 106 and/or the front side panels 108 and 110 may also be folded laterally inward toward the inner surfaces 132 of the waist regions 116 and 118.

The absorbent article 100 may also include various configurations of fastening elements to enable fastening of the front waist region 116 and the back waist region 118 together to form a closed waist circumference and leg openings once the absorbent article is positioned on a wearer. For example, as shown in Figures 1A and 1B, the absorbent article 100 may include first and second fastening members 162, 164, also referred to as tabs, connected with the first and second rear side panels 104, 106, respectively. The absorbent article may also include first and second front side panels 108, 110, that may or may not include fastening members.

With continued reference to Figures 1A and 1B, each side panel 104, 106 and/or fastening member 162 and 164 may form a portion of or may be permanently bonded, adhered or otherwise joined directly or indirectly to the chassis 102 laterally inward from the side edge 128 and 130, in one of the front waist region 116 or the back waist region 118. Alternatively, the fastening members 162, 164 may form a portion of or may be permanently bonded, adhered or otherwise joined directly or indirectly to the first and second rear panels 104, 106 at or adjacent the distal edge of the panel and/or the first and second front side panels 108 and 110 at or adjacent the distal edge of the side panel. It is to be appreciated that the fastening members and/or side panels may be assembled in various ways, such as disclosed for example, in U.S. Patent No. 7,371,302, which is incorporated by reference herein. The fastening members 162, 164 and/or side panels 104, 106, 108, 110 may also be permanently bonded or joined at or adjacent the side edges 128 and 130 of the chassis 102 in various ways, such as for example, by adhesive bonds, sonic bonds, pressure bonds, thermal bonds or combinations thereof, such as disclosed for example, U.S. Patent No. 5,702,551

Referring now to Figure 1B, the first fastening member 162 and/or the second fastening member 164 may include various types of releasably engageable fasteners. The first and second fastening members 162 and/or 164 may also include various types of refastenable fastening structures. For example, the first and second fastening members 162 and 164 may include mechanical fasteners, 166, in the form of hook and loop fasteners, hook and hook fasteners, macrofasteners, buttons, snaps, tab and slot fasteners, tape fasteners, adhesive fasteners, cohesive fasteners, magnetic fasteners, hermaphroditic fasteners, and the like. Some examples of fastening systems and/or fastening members 162, 164 are discussed in U.S. Patent Nos. 3,848,594; 4,662,875; 4,846,815; 4,894,060; 4,946,527; 5,151,092; 5,221,274; 5,242,436; 6,251,097; 6,669,618; 6,432,098; U.S. Pub. Nos. 2007/0078427 A1 and 2007/0093769 A1; and U.S. Patent Application No. 16/685,230,

As previously mentioned, the fastening members 162 and 164 may be constructed from various materials and may be constructed as a laminate structure. The fastening members 162 and 164 may also be adapted to releasably and/or refastenably engage or connect with another portion of the absorbent article 100. For example, as shown in Figure 1A, the absorbent article 100 may include a connection zone 168, sometimes referred to as a landing zone, in the first waist region 116. As such, when the taped absorbent article 100 is placed on a wearer, the fastening members 162 and 164 may be pulled around the waist of the wearer and connected with the connection zone 168 in the first waist region 116 to form a closed waist circumference and a pair of laterally opposing leg openings. It is to be appreciated that the connection zone may be constructed from a separate substrate that is connected with the chassis 102 of the absorbent article. In some embodiments, the connection zone may be integrally formed as part of the backsheet 136 of the absorbent article 100 or may be formed as part of the first and second front panels 108, 110, such as described in U.S. Patent Nos. 5,735,840 and 5,928,21

With continued reference to Figure 1B, the absorbent article 100 may also include leg gasketing elements 156. It is to be appreciated that the leg gasketing elements 156 can be and are sometimes also referred to as leg cuffs, leg bands, side flaps, barrier cuffs, elastic cuffs or gasketing cuffs. The leg gasketing elements 156 may be elasticized and may be configured in various ways to help reduce the leakage of body exudates in the leg regions. Example leg gasketing elements 156 may include those described in U.S. Patent Nos. 3,860,003; 4,909,803; 4,695,278; 4,795,454; 4,704,115; and U.S. Pub. No. 2009/0312730 A1,

As shown in Figure 1B, the absorbent article 100 may include longitudinally extending and laterally opposing leg gasketing elements 156 that are disposed on the interior surface 132 of the chassis 102 that faces inwardly toward the wearer and contacts the wearer. Each leg gasketing element 156 may have a first side edge 157 and a second side edge 159, wherein the first side edge 157 is positioned laterally inboard of the second side edge 159. The leg gasketing elements 156 may also overlap the absorbent assembly 140, wherein the first side edges 157 extend laterally inward of the respective side edges 152, 154 of the absorbent assembly 140. In some configurations, the leg gasketing elements 156 may not overlap the absorbent assembly 140. It is to be appreciated that the leg gasketing elements 156 may be formed in various ways, such as for example, by folding portions of the chassis 102 laterally inward, i.e., toward the longitudinal axis 124, to form both the respective leg gasketing elements and the side edges 128 and 130 of the chassis 102. In another example, the leg gasketing elements 156 may be formed by attaching an additional layer or layers to the chassis 102 at or adjacent to each of the respective side edges and of the chassis. Each of the leg gasketing elements 156 may be joined to the interior surface 132 of the chassis and/or the absorbent assembly 140 in leg gasketing element attachment zones in the front waist region 116 and in leg gasketing element attachment zones in the back waist region 118. The leg gasketing elements 156 may extend to the same longitudinal extent as the absorbent article 100 or alternatively the leg gasketing elements 156 may have a longitudinal extent that is less than the absorbent article 100. **In** some configurations, the leg gasketing elements may be configured to define inner cuffs, outer cuffs, or both inner and outer cuffs.

The absorbent article 100 may also include one or more waist panels 158, such as shown in Figure 1B. The waist panel 158 may provide improved fit and containment and may define a portion or zone of the absorbent article 100 that may elastically expand and contract to dynamically fit a wearer's waist. The absorbent article 100 may also include more than one waist panels 158, for example, having a first panel 158a positioned in the first waist region 116 and second panel 158b positioned in the second waist region 118, although other configurations may be constructed with a single waist panel 158. The waist panel 158 may be constructed in a number of different configurations including those described in U.S. Patent Nos. 4,515,595 and 5,151,092, and U.S. Pub. Nos. 2003/75807 A1; 2020/0375815 A1; 2021/0128366 A1; and 2021/0128369 A1,

It is to be appreciated that the waist panels 158 herein may be configured in various ways and may include one or more elastic materials, such as for example, elastic film and/or strands. For example, the waist panel 158 may be configured as a single layer of elastic film. In some configurations, the waist panel 158 may be configured as a laminate of two more substrates. For example, the waist panel 158 may be configured as an elastic film bonded in between two or more nonwoven substrates and/or may be bonded with one or more nonwoven substrates. For example, the waist panel 158 may be configured as a bi-laminate with an elastic film bonded with a single nonwoven substrate. In another example, the waist panel 158 may be configured as an elastic film bonded between two or more substrates, wherein the substrates may comprise nonwovens. It is also to be appreciated that nonwoven substrates of the waist panel 158 may be of the same or different material and/or basis weights and may be configured as an elastomeric nonwoven or a non-elastic nonwoven. In some configurations, one more nonwoven substrates of the waist panel 158 may be of the same or different material and/or basis weights as one more nonwoven substrates of the topsheet 138, backsheet 136, and/or leg gasketing elements 156.

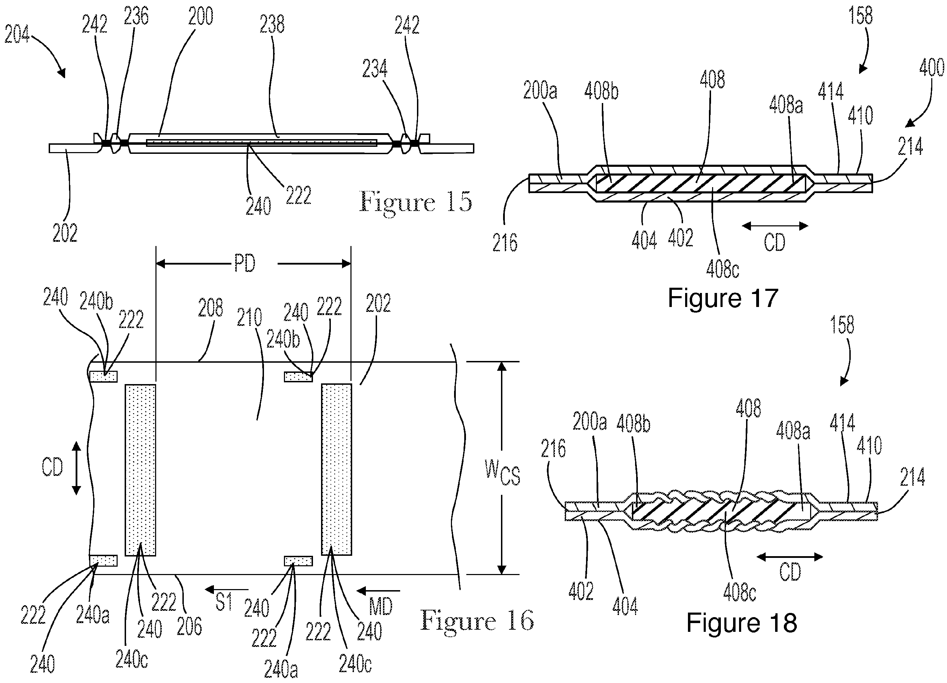

It is to be appreciated that the waist panels 158 herein may be formed in various ways and may include various components bonded together in various ways and with differing or identical bond patterns. For example, the waist panels 158 herein may comprise a laminate of an elastic film bonded with at least one nonwoven in a stretched state. For example, Figures 17 and 18 show cross sectional views of a waist panel 158 configured as a laminate 400 that includes a first substrate 402, a second substrate 410, and an elastic film 408 positioned between the first substrate 402 and the second substrate 410, wherein the first substrate 402 and/or second substrate 410 may be configured as a nonwoven as discussed above. In some configurations, the laminate may be bonded continuously or discontinuously. In some configurations, the laminate may be bonded with a plurality of individual bond sites that may or may not form a visually discernable pattern. The first substrate 402 and the second substrate 410 of the waist panel 158 may be the same or different types of nonwovens and/or may have the same or different basis weights. In addition, the chassis 102 may include one or more nonwoven substrates. As such, the first substrate 402 and/or the second substrate 410 of the waist panel 158 may be the same or different types of nonwovens and/or may have the same or different basis weights as a nonwoven substrate of the chassis 102. In addition, the nonwoven substrates of waist panel 158, such as the first substrate 402 and/or the second substrate 410 for example, may include nonwoven substrates having the same or different fiber orientations as a nonwoven substrate of the chassis 102. In turn, the waist panel 158 and a topsheet or backsheet in an absorbent article may each include nonwoven substrates that are the same or different types of nonwovens and/or may have the same or different basis weights and/or may have the same or different fiber orientations.

It is to be appreciated that components of the waist panel 158 may be bonded together in various ways, such as for example, by adhesive bonds, ultrasonic bonds, pressure bonds, thermal bonds or combinations thereof. It is to be appreciated that components of the waist panel 158 may be bonded together with adhesive applied in various ways, such as for example, as a spray nozzle and/or a slot coating device. In some configurations, components of the waist panel 158 may be continuously bonded with adhesive or bonded discontinuously with a patterned adhesive. In some configurations, the adhesive may be applied in accordance with the apparatuses and/or methods disclosed in U.S. Patent Nos. 8,186,296; 9,265,672; 9,248,054; and 9,295,590 and U.S. Pub. No. 2014/0148773 A1, In some configurations, components of the waist panel 158 may be mechanically (pressure) bonded with the application of pressure (and optionally heat) in various ways, such as for example, the mechanical bonding devices and methods disclosed in in U.S. Patent Nos. 4,854,984; 6,248,195; 8,778,127; 9,005,392; 9,962,297; and 10,052,237, In some configurations, components of the waist panel 158 may be mechanically (pressure) bonded with the use of ultrasonic bonding methods configured in various ways, such as for example linear or rotary type configurations, and such as disclosed for example in U.S. Patent Nos. 3,113,225; 3,562,041; 3,733,238; 5,110,403; 6,036,796; 6,508,641; and 6,645,330.

In some configurations, the elastic film 408 may be bonded together with the first and/or second substrates 402, 410, and the first substrate 402 may be bonded directly to the second substrate 410 in areas of the waist panel 158. In some configurations, the first and second substrates 402, 410 may be bonded directly to each other through apertures in the elastic film 408, wherein such apertures may be formed during the bonding process. In some configurations, the elastic film 408 can be involved, or participate, in the bonding between the first and second substrates 402, 410, wherein "involved" can mean that the elastic film 408 can, to some extent, be in intimate contact with, and possibly partially merged with, one or both the first and second substrates 402, 410. The involvement may be due to actual melt bonding about the perimeter of a bond site or may be due to mechanical interaction, such as by entanglement of a fibrous elastic layer between fibrous nonwoven layers also about the perimeter of bond site. It is to be appreciated that the waist panel 158 may be formed with various types of bond configurations, such as disclosed, for example, in U.S. Patent Nos. 6,572,595; 6,830,800; 7,087,287; and 7,803,244; and U.S. Pub. Nos. 2018/0042778 A1; 2018/0042787 A1; 2018/0042779 A1; and 2018/0042780 A1,

In some configurations, the waist panel 158 may be formed as a zero strain stretch laminate that may be connected with the chassis 102 in a stretched state. In some configurations, the zero strain stretch laminate may include at least a layer of nonwoven material and an elastomeric element. The elastomeric element may be attached to the layer of nonwoven material while in a relaxed or substantially relaxed state, and the resulting laminate is made stretchable (or more stretchable over a further range) by subjecting the laminate to an activation process, which elongates the nonwoven layer permanently and elongates the elastomeric element temporarily. In some configurations, the nonwoven layer may be a separate component, in which case the elastomeric element is attached to the nonwoven layer to form the laminate, which is then connected with the chassis 102. In some configurations, the nonwoven layer may be integral with at least a portion of the chassis 102, in which case the elastomeric element may be attached to the nonwoven layer and the nonwoven/elastomeric element laminate is subsequently activated. In some configurations, the waist panel may be an extrusion bonded laminate. If one or more layers of the waist panel 158 are provided separately, the waist panel 158 may be activated either before or after attachment to the chassis 102. Examples of zero strain activation processes are disclosed in U.S. Patent Nos. 5,167,897 and 5,156,793

It is to be appreciated that the waist panel 158 may be located in various positions relative to the garment facing surfaces 132 and wearer facing surfaces 134 of various absorbent article components. In some configurations, the waist panel 158 may be positioned on the wearer facing surface 132 of the topsheet 138. In some configurations, the waist panel 158 may be positioned on the wearer facing surfaces 132 of the topsheet 138 and the leg gasketing elements 156. In some configurations, the waist panel 158 may be positioned on the wearer facing surfaces 132 of the topsheet 138 and laterally opposing end regions of the waist panel 158 may be positioned between the leg gasketing elements 156 and the topsheet 138. In some configurations, the waist panel 158 may be positioned between the garment facing surface 132 of the topsheet 138 and the wearer facing surface 132 of the backsheet 136. And in some configurations, the waist panel 158 may be positioned on the garment facing surface 134 of the backsheet 136.

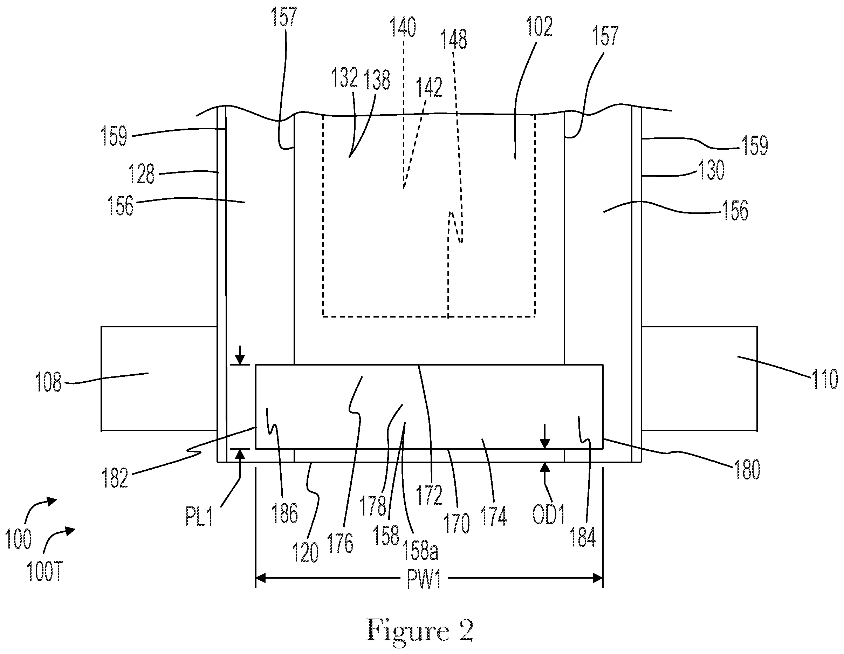

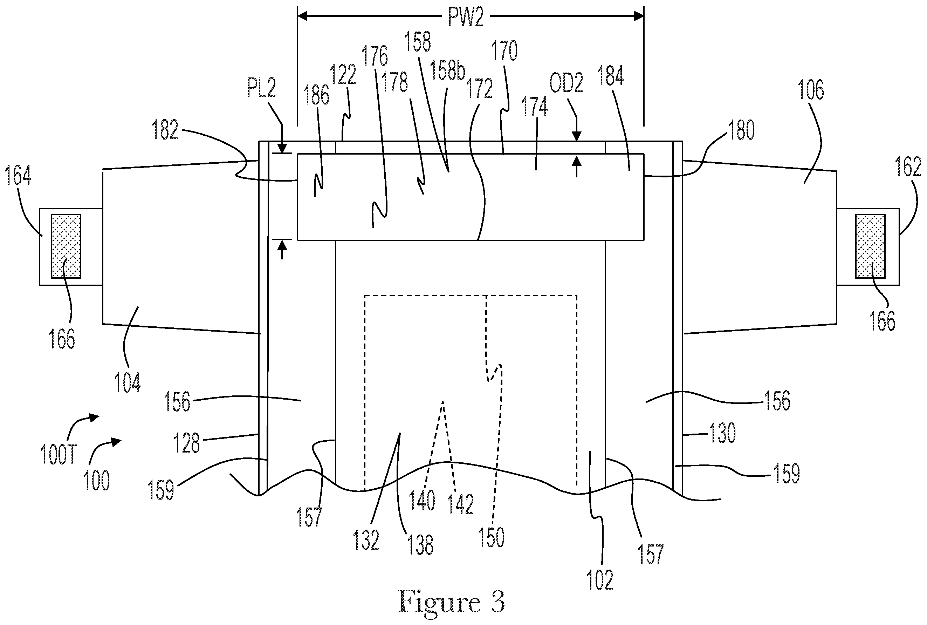

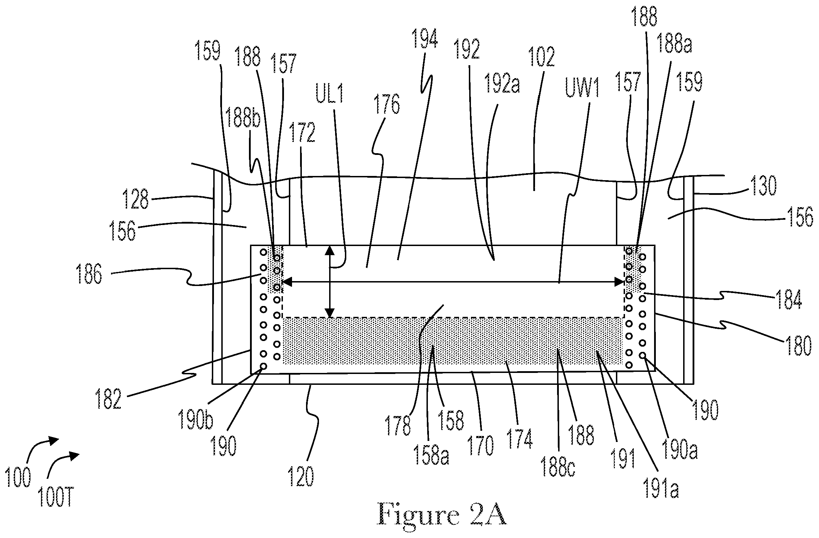

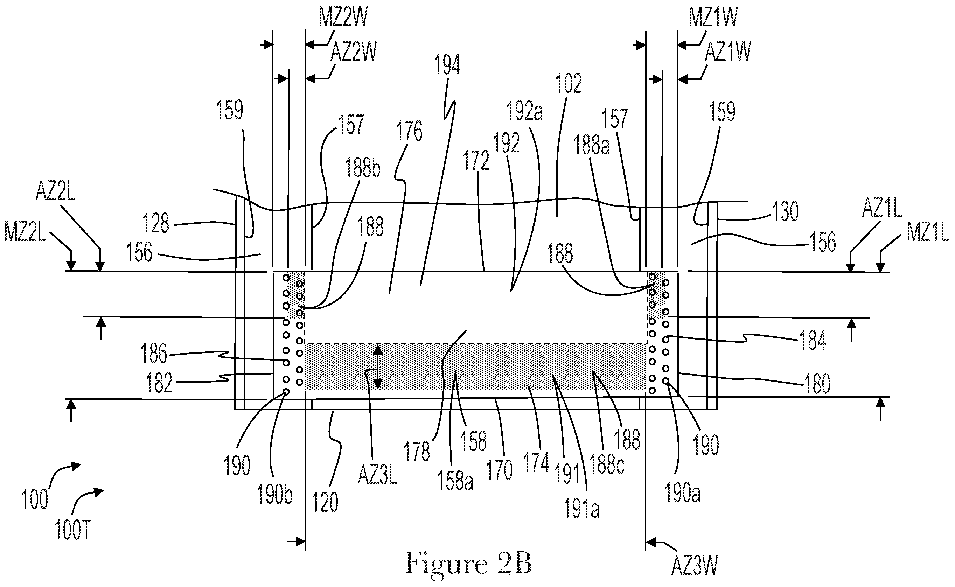

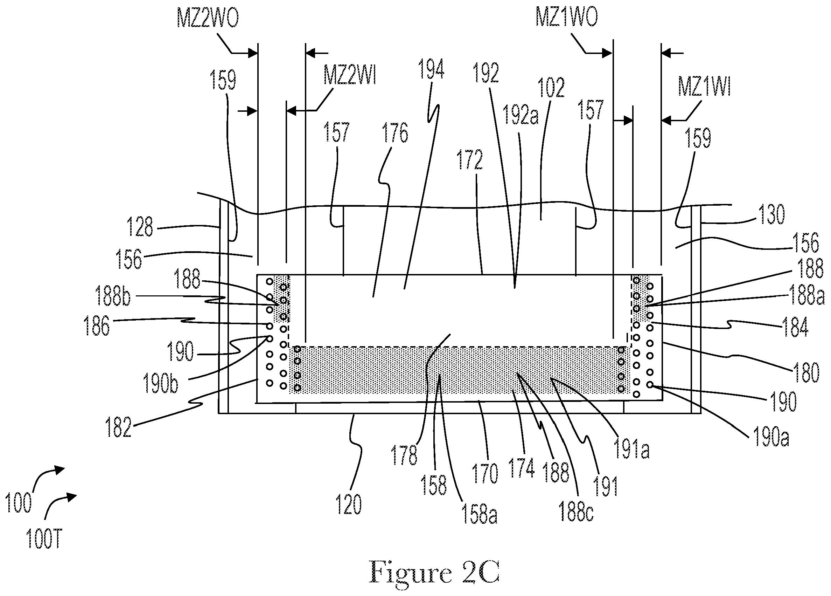

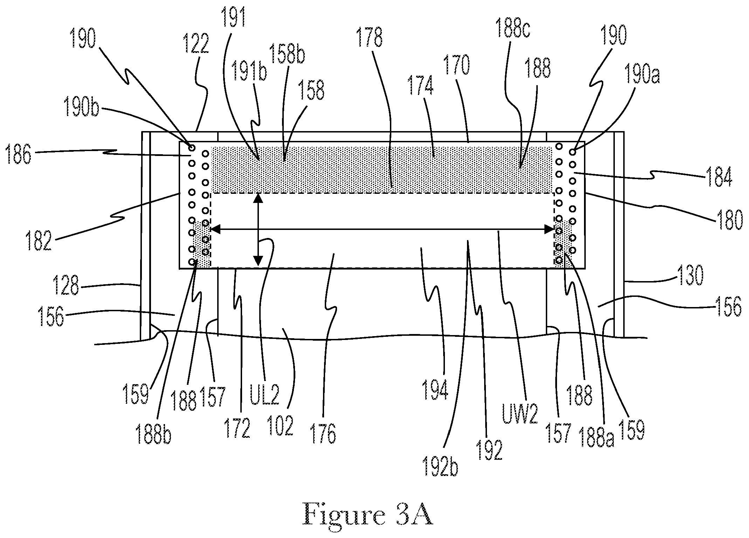

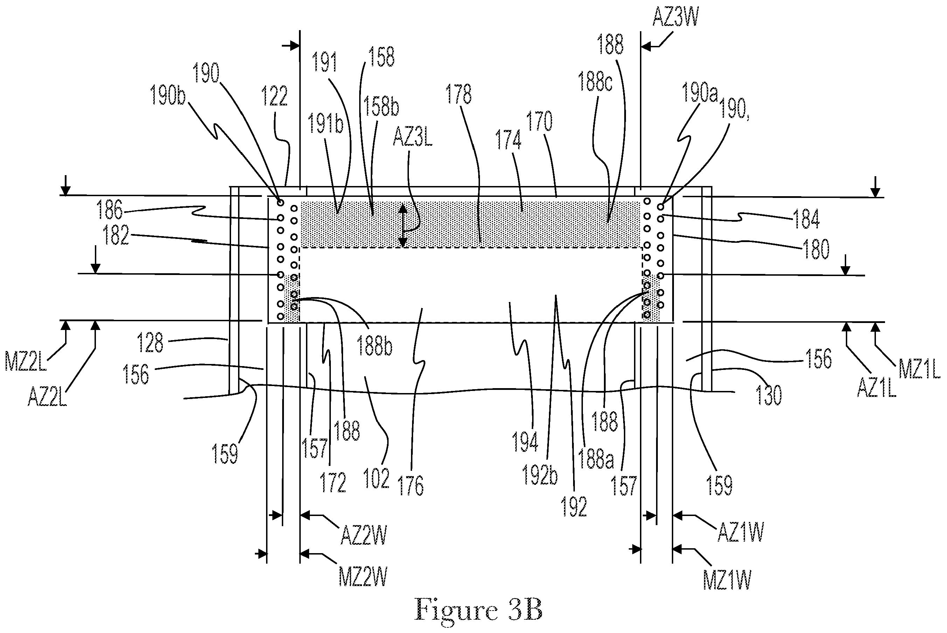

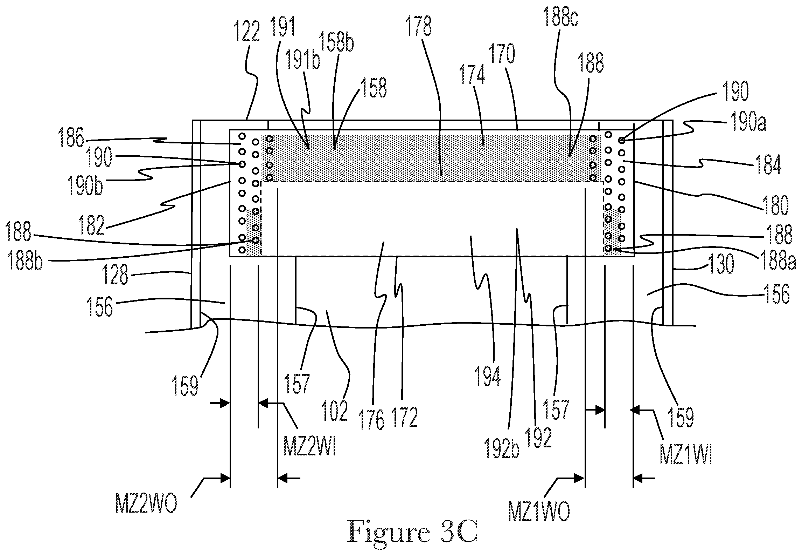

As shown in Figures 2 and 3, the first and second waist panels 158a, 158b herein may each comprise a first lateral edge 170 and a second lateral edge 172, wherein the second lateral edge 172 is positioned longitudinally inward relative the first lateral edge 170. As such, the first lateral edge 170 may be configured as an outboard lateral edge, and the second lateral edge 172 may be configured as an inboard lateral edge. In addition, the first and second waist panels 158a, 158b may comprise a first longitudinal end region 174 adjacent the first lateral edge 170 and a second longitudinal end region 176 adjacent the second lateral edge 172, wherein the first and second longitudinal end regions 174, 176 are separated by a central region 178. The first and second lateral edges 170, 172 may be connected with and separated by a first longitudinal edge 180 and a second longitudinal edge 182. As such, the first and second waist panels 158a, 158b may also include a first lateral end region 184 adjacent the first longitudinal edge 180 and a second lateral end region 186 adjacent the second longitudinal edge 182, wherein the first and second lateral end regions 184, 186 are separated by the central region 178. In some configurations, the first lateral edge 170, second lateral edge 172, first longitudinal edge 180, and/or second longitudinal edge 182 may be defined by a fold line, wherein one or more layers of waist panel 158 may have been folded onto itself or another layer during assembly. In some configurations, the first lateral edge 170, second lateral edge 172, first longitudinal edge 180, and/or second longitudinal edge 182 may be defined by unfolded edge or a cut line, wherein one or more layers of waist panel 158 may have been cut or trimmed during assembly.

As discussed above, the waist panels 158 herein may be elastic and may comprise at least one direction of stretch. In some configurations, the direction of stretch may be laterally oriented between the first longitudinal edge 180 and the second longitudinal edge 182. In some configurations, the first waist panel 158a and/or the second waist panel 158b may be configured to extend at least about 10 mm with an applied force greater than 0 to about 3N. It is also to be appreciated that the first waist panel 158a may comprise stretch characteristics that are the same or different from stretch characteristics of the second waist panel 158b. Such stretch characteristics may comprise a percent contraction or a percent elongation. In some configurations, the stretch characteristics of the first waist panel 158a may be the same or may vary between the first lateral edge 170 and the second lateral edge 172 and/or the between the first longitudinal edge 180 and the second longitudinal edge 182. And in some configurations, the stretch characteristics of the second waist panel 158b may be the same or may vary between the first lateral edge 170 and the second lateral edge 172 and/or the between the first longitudinal edge 180 and the second longitudinal edge 182.

It is to be appreciated that the waist panels 158 herein may be configured with various shapes and/or sizes. For example, as shown in Figures 2 and 3, the first waist panel 158a may comprise a first width PW1 extending between first and second longitudinal edges 180, 182, and the second waist panel 158b may comprise a second width PW2 extending between first and second longitudinal edges 180, 182. It is to be appreciated that the first width PW1 and the second width PW2 may be equal or different. In some configurations, the first width PW1 and/or the second width PW2 may be from about 80 mm to about 250 mm, specifically reciting all 1 mm increments within the above-recited ranges and all ranges formed therein or thereby. The first waist panel 158a may comprise a first length PL1 extending between first and second lateral edges 170, 172, and the second waist panel 158b may comprise a second length PL2 extending between first and second lateral edges 170, 172. It is to be appreciated that the first length PL1 and the second length PL2 may be equal or different. In some configurations, the first length PL1 and/or the second length PL2 may be from about 5 mm to about 80 mm, specifically reciting all 1 mm increments within the above-recited ranges and all ranges formed therein or thereby.

It is to be appreciated that the waist panels 158 may be located in various lateral and longitudinal positions relative to various absorbent article components. In some configurations, the waist panel 158 may be positioned such that the first and second longitudinal edges 180, 182 of the waist panel 158 are located laterally inboard of the leg gasketing elements 156. In some configurations, the waist panel 158 may be positioned such that the first and second longitudinal edges 180, 182 and the first and second longitudinal end regions 174, 176 of the waist panel 158 overlap the leg gasketing elements 156. In some configurations, the first waist panel 158a may be positioned longitudinally inboard from the first waist edge 120 of the absorbent article 100 and/or toward or overlapping the first lateral edge 148 of the absorbent assembly 140; and the second waist panel 158b may be positioned longitudinally inboard from the second waist edge 122 of the absorbent article 100 and/or toward or overlapping the second lateral edge 150 of the absorbent assembly 140. In some configurations, the first waist panel 158a and/or the second waist panel 158b may overlap the first lateral edge 148 and/or second lateral edge 150 of the absorbent assembly 140 from about 1mm to about 45mm, specifically reciting all 1 mm increments within the above-recited ranges and all ranges formed therein or thereby. In some configurations, the first lateral edge 170 of the first waist panel 158a may be positioned longitudinally inboard from the first waist edge 120 by an offset distance OD1 that is greater than zero. In some configurations, the first lateral edge 170 of the second waist panel 158b may be positioned longitudinally inboard from the second waist edge 122 by an offset distance OD2 that is greater than zero. In some configurations, the offset distance OD1 and/or the offset distance OD2 may be at least 5 mm. In some configurations, the first lateral edge 170 of the first waist panel 158a may be coterminous with the first waist edge 120 such that the offset distance OD1 is zero. In some configurations, the first lateral edge 170 of the second waist panel 158b may be coterminous with the second waist edge 122 such that the offset distance OD2 is zero.

It is to be appreciated that the first waist panel 158a and/or the second waist panel 158b may be bonded with the chassis 102 and/or leg gasketing elements 156 in various ways, such as for example, by adhesive bonds, ultrasonic bonds, pressure bonds, thermal bonds or combinations thereof. It is to be appreciated that the first waist panel 158a and/or the second waist panel 158b may be bonded with the chassis 102 and/or leg gasketing elements 156 with adhesive applied in various ways, such as for example, as a spray nozzle and/or a slot coating device. In some configurations, the first waist panel 158a and/or the second waist panel 158b may be continuously bonded with the chassis 102 and/or leg gasketing elements 156 with adhesive or bonded discontinuously with a patterned adhesive. In some configurations, the adhesive may be applied in accordance with the apparatuses and/or methods disclosed in U.S. Patent Nos. 8,186,296; 9,265,672; 9,248,054; and 9,295,590 and U.S. Pub. No. 2014/0148773 A1, In some configurations, the first waist panel 158a and/or the second waist panel 158b may be mechanically (pressure) bonded with the chassis 102 and/or leg gasketing elements 156 with the application of pressure (and optionally heat) in various ways, such as for example, the mechanical bonding devices and methods disclosed in in U.S. Patent Nos. 4,854,984; 6,248,195; 8,778,127; 9,005,392; 9,962,297; and 10,052,237, In some configurations, the first waist panel 158a and/or the second waist panel 158b may be mechanically (pressure) bonded with the chassis 102 and/or leg gasketing elements 156 with the use of ultrasonic bonding methods configured in various ways, such as for example linear or rotary type configurations, and such as disclosed for example in U.S. Patent Nos. 3,113,225; 3,562,041; 3,733,238; 5,110,403; 6,036,796; 6,508,641; and 6,645,330.

As previously mentioned, it is to be appreciated that the waist panels 158 herein may be bonded with the chassis 102 and/or leg gasketing elements 156 with combinations of adhesive bonds and pressure bonds. For example, as shown in Figure 2A, the first longitudinal end region 174 of the first waist panel 158a may be bonded with the chassis 102 and/or leg gasketing elements 156 with adhesive bonds 188, which are generically illustrated by shaded regions. In addition, the first and second lateral end regions 184, 186 of the first waist panel 158a may be bonded with the chassis 102 and/or leg gasketing elements 156 with pressure bonds 190. In some configurations, the first and second lateral end regions 184, 186 of the first waist panel 158a may be bonded with inner cuffs and/or outer cuffs of leg gasketing elements 156. As shown in Figure 3A, the first longitudinal end region 174 of the second waist panel 158b may be bonded with the chassis 102 and/or leg gasketing elements 156 with adhesive bonds 188, which are generically represented by shaded regions.

In addition, the first and second lateral end regions 184, 186 of the second waist panel 158b may be bonded with the chassis 102 and/or leg gasketing elements 156 with pressure bonds 190. In some configurations, the first and second lateral end regions 184, 186 of the second waist panel 158b may be bonded with inner cuffs and/or outer cuffs of leg gasketing elements 156. In some configurations, the pressure bonds 190 may be a discontinuous pattern of discrete bond sites. It is to be appreciated that the discrete bond sites may define various sizes and shapes and may be separated from each other by various distances. For example, in some configurations, the discrete bond sites may be separated from each other by at least 0.2 mm. It is also to be appreciated that the discrete bond sites may cover various different sized areas of the waist panel. For example, in some configurations, the plurality of discrete bond sites may comprise from about 5% to about 50% of an area of the waist panel. In some configurations, the first and second lateral end regions 184, 186 extending along the first and second longitudinal edges 180, 182 may be bonded with the chassis 102 and/or leg gasketing elements 156 with a continuous bond that defines a sealed edge.

In some configurations, one or more regions of the waist panel 158 (referred to herein as bonded regions 191) may be bonded with the chassis 102 and/or leg gasketing elements 156, and one or more regions of the waist panel 158 (referred to as unbonded regions 192) may not be bonded (unattached) with the chassis 102 and/or leg gasketing elements 156, thereby forming a pocket 194 between the waist panel 158 and the chassis 102. For example, as shown in Figure 2A, the first waist panel 158a may comprise bonded regions 191a wherein the first longitudinal end region 174, the first lateral end region 184, and the second lateral end region 186 of the first waist panel 158a are bonded with chassis 102 and/or leg gasketing elements 156; and the first waist panel 158a may comprise at least one unbonded region 192a (generically illustrated by a rectangle with a dashed border) wherein a portion of the second longitudinal end region 176 and at least a portion the second lateral edge 172 may be unattached to the chassis 102 and/or leg gasketing elements 156. With continued reference to Figure 3A, the second waist panel 158b may comprise bonded regions 191b wherein the first longitudinal end region 174, the first lateral end region 184, and the second lateral end region 186 of the second waist panel 158b are bonded with chassis 102 and/or leg gasketing elements 156; and the second waist panel 158b may comprise at least one unbonded region 192b (generically illustrated by a rectangle with a dashed border) wherein a portion of the second longitudinal end region 176 and at least a portion the second lateral edge 172 may be unattached to the chassis 102 and/or leg gasketing elements 156.

It is to be appreciated that the waist panels 158 herein may be configured with one or more unbonded regions with various shapes and/or sizes. For example, as shown in Figures 2A and 3A, the first waist panel 158a may comprise a first unbonded region 192a and/or the second waist panel 158b may comprise a second unbonded region 192b. As such, the first unbonded region 192a may comprise a laterally extending first width UW1 and a longitudinally extending first length UL1, and the second unbonded region 192b may comprise a laterally extending second width UW2 and a longitudinally extending second length UL2. It is to be appreciated that the first width UW1 and the second width UW2 may be equal or different. In some configurations, the first width UW1 and/or the second width UW2 may be from about 40 mm to about 200 mm, specifically reciting all 1 mm increments within the above-recited ranges and all ranges formed therein or thereby. It is also to be appreciated that the first length UL1 and the second length UL2 may be equal or different. In some configurations, the first length UL1 and/or the second length UL2 may be from about 10 mm to about 50 mm, specifically reciting all 1 mm increments within the above-recited ranges and all ranges formed therein or thereby. In some configurations, the first unbonded region 192a may comprise a first area A1 and/or the second unbonded region 192b may comprise a second area A2, wherein the first area A1 and the second area may be equal or different. In some configurations, the first area A1 and/or the second area A2 may be from about 400 mm² to about 10000 mm², specifically reciting all 1 mm² increments within the above-recited ranges and all ranges formed therein or thereby. It is to be appreciated that the waist panels 158 may also be bonded to absorbent article components with permanent and frangible bond arrangements, such as disclosed in U.S. Patent Application No. 63/208,019,

It is to be appreciated that the adhesive bonds 188 and/or the mechanical bonds 190 discussed above with reference the first waist panel 158a and/or the second waist panel 158b in Figures 2A and 3A may be configured in various ways so to define various zones having different shapes and sizes. For example, as shown in Figures 2A-2C and 3A-3C the adhesive bonds 188 may be arranged to define a first zone 188a of adhesive 188; a second zone 188b of adhesive 188; and a third zone 188c of adhesive 188. In addition, the pressure bonds 190 may be arranged to define a first zone 190a of mechanical bonds 190 and a second zone 190b of mechanical bonds 190. It is to be appreciated that the same or different types of adhesives may be applied in the first zone 188a, the second zone 188b, and the third zone 188c of adhesive 188. It is also to be appreciated that adhesive may applied in the same or different manners, such as with spray nozzle and/or a slot coating device, in the first zone 188a, the second zone 188b, and the third zone 188c of adhesive 188.

As shown in Figures 2A-2C and 3A-3C, the first zone 188a of adhesive 188 may be positioned in the first lateral end region 184 and adjacent the inboard lateral edge 172 of the waist panel 158; and the second zone 188b of adhesive 188 may be positioned in the second lateral end region 186 and adjacent the inboard lateral edge 172 of the waist panel 158. The first zone 188a of adhesive 188 and/or the second zone 188b of adhesive 188 may bond the waist panel 158 with at least one of the chassis 102 and the leg gasketing element 156. The third zone 188c of adhesive 188 may be positioned in the first longitudinal end region 174 and may be adjacent the outboard lateral edge 170 of the waist panel 158 and may extend laterally through the central region 178. The third zone 188c of adhesive 188 may bond the waist panel 158 with the chassis 102 and/or leg gasketing elements 156.

With continued reference to Figures 2A-2C and 3A-3C, the first zone 190a of mechanical bonds 190 may be positioned in the first lateral end region 184 and may extend between the outboard lateral edge 170 and the inboard lateral edge 172 of the waist panel 158; and the second zone 190b of mechanical bonds 190 may be positioned in the second lateral end region 186 and may extend between the outboard lateral edge 170 and the inboard lateral edge 172 of the waist panel 158. The first zone 190a of mechanical bonds 190 and/or the second zone 190b of mechanical bonds 190 may bonding the waist panel 158 with at least one of the chassis 102 and the leg gasketing elements 156.

It is to be appreciated that the first zone 188a, second zone 188b, and/or third zone 188c of adhesive 188 may be positioned in various ways relative to the first zone 190a of mechanical bonds 190 and/or the second zone 190b of mechanical bonds 190. For example, in some configurations, the first zone 188a and/or third zone 188c of adhesive 188 may have portions that overlap with the first zone 190a of mechanical bonds 190; and the second zone 188b and/or third zone 188c of adhesive 188 may have portions that overlap with the second zone 190b of mechanical bonds 190. **In** some configurations, the first zone 188a and/or third zone 188c of adhesive 188 may be completely separated from or partially border the first zone 190a of mechanical bonds 190; and the second zone 188b and/or third zone 188c of adhesive 188 may be completely separated from or partially border the second zone 190b of mechanical bonds 190. For example, as shown in Figures 2B and 3B, the first zone 188a of adhesive 188 may overlap with the first zone 190a of mechanical bonds 190; and the second zone 188b of adhesive 188 may overlap with the second zone 190b of mechanical bonds 190. **In** another example, as shown in Figures 2C and 3C, the third zone 188c of adhesive 188 may overlap with the first zone 190a of mechanical bonds 190 and the second zone 190b of mechanical bonds 190.

It is also to be appreciated that the first zone 188a, second zone 188b, and/or third zone 188c of adhesive 188 may have various different shapes and sizes; and the first zone 190a of mechanical bonds 190 and/or the second zone 190b of mechanical bonds 190 may have various different shapes and sizes. As shown for example, in Figures 2B and 3B, the first zone 190a of mechanical bonds 190 may comprise a lateral width MZ1W and the first zone 188a of adhesive 188 may comprise a lateral width AZ1W; and the second zone 190b of mechanical bonds 190 may comprise a lateral width MZ2W and the second zone 188b of adhesive 188 may comprise a lateral width AZ2W. In some configurations, the lateral width MZ1W is greater than the lateral width AZ1W, and/or the lateral width MZ2W is greater than the lateral width AZ2W. In some configurations, the lateral width MZ1W may be equal to or less than the lateral width AZ1W, and/or the lateral width MZ2W may be less than or equal to the lateral width AZ2W. The third zone 188c of adhesive 188 may comprise a lateral width AZ3W that is equal to or less than the first width PW1 of the first waist panel 158a and/or less than or equal to the second width PW2 of the second waist panel 158b.

With continued reference to Figures 2B and 3B, the first zone 190a of mechanical bonds 190 may comprise a longitudinal length MZ1L and the first zone 188a of adhesive 188 may comprise a longitudinal length AZ1L; and the second zone 190b of mechanical bonds 190 may comprise a longitudinal length MZ2L and the second zone 188b of adhesive 188 may comprise a longitudinal length AZ2L. In some configurations, the longitudinal length MZ1L is greater than the longitudinal length AZ1L, and/or the longitudinal length MZ2L is greater than the longitudinal length AZ2L. In some configurations, the longitudinal length MZ1L may be equal to or less than the longitudinal length AZ1L, and/or the longitudinal length MZ2L may be less than or equal to the longitudinal length AZ2L. In some configurations, the longitudinal length AZ1L and/or the longitudinal length AZ2L may be equal to or less than half the of the first waist panel 158a and/or equal or less than half the second length PL2 of the second waist panel 158b. In some configurations, the longitudinal length MZ1L, the longitudinal length AZ1L, the longitudinal length MZ2L, and/or the longitudinal length AZ2L may be equal to or less than the first length PL1 of the first waist panel 158a and/or equal or less than the second length PL2 of the second waist panel 158b. The third zone 188c of adhesive 188 may comprise a longitudinal length AZ3L that is less than or equal to the first length PL1 of the first waist panel 158a and/or less than or equal to the second length PL2 of the second waist panel 158b.

In some configurations, the first zone 188a of adhesive 188 and/or the second zone 188b of adhesive 188 may be coterminous with the inboard lateral edge 172 of the waist panel 158 and/or may be positioned longitudinally outboard of the inboard lateral edge 172 of the waist panel 158. In some configurations, the first zone 188a of adhesive 188 and/or the second zone 188b of adhesive 188 may be coterminous with the inboard lateral edge 172 and/or the outboard lateral edge 172 of the waist panel 158. In some configurations, the first zone 188a of adhesive 188 and/or the second zone 188b of adhesive 188 may be longitudinally offset from the inboard lateral edge 172 and/or the outboard lateral edge 172 of the waist panel 158. In some configurations, the first zone 188a of adhesive 188 and/or the first zone 190a of mechanical bonds 190 may be coterminous with or laterally offset from the first longitudinal edge 180 of the waist panel 158; and/or the second zone 188b of adhesive 188 and/or the second zone 190b of mechanical bonds 190 may be coterminous with or laterally offset from the second longitudinal edge 182 of the waist panel 158. In some configurations, the third zone 188c of adhesive 188 may be conterminous with or longitudinally inboard of the outboard edge 170 of the waist panel 158 and/or may be conterminous with or laterally offset from the first longitudinal edge 180 and/or the second longitudinal edge 182 of the waist panel 158.

It is to be appreciated that the lateral widths of the first zone 188a, second zone 188b, and/or third zone 188c of adhesive and/or the lateral widths of the first zone 190a and/or second zone of mechanical bonds 190 may vary along longitudinal lengths. For example, as shown in Figures 2C and 3C, the lateral width MZ1W of the first zone 190a of mechanical bonds 190 and/or the lateral width MZ2W of the second zone 190b of mechanical bonds 190 may vary. As illustrated, the first zone 190a of mechanical bonds 190 may comprise a first lateral width MZ1WI and a second lateral width MZ1WO that is longitudinally outboard of the first lateral width MZ1WI; and the second zone 190b of mechanical bonds 190 may comprise a first lateral width MZ2WI and a second lateral width MZ2WO that is longitudinally outboard of the first lateral width MZ2WI. In some configurations, the first lateral width MZ1WI may be less than the second lateral width MZ1WO, and/or the first lateral width MZ2WI may be less than the second lateral width MZ2WO. In configurations, the first lateral width MZ1WI may be greater than or equal to the second lateral width MZ1WO, and/or the first lateral width MZ2WI may be greater than or equal to the second lateral width MZ2WO.

It is to be appreciated that the overlapping of the first, second, and/or third zones 188a, 188b, 188c of adhesive 188 with the first and/or second zones 190a, 190b of mechanical bonds 190 and/or the positioning of the first and second zones 188a, 188b of adhesive on or adjacent the inboard edge 172 of the waist panels 158 may help to reinforce and/or strengthen bonded regions. Such reinforcements may help prevent and/or reduce instances of separation of the waist panel 158 from other absorbent article from the topsheet 138 and/or leg gasketing elements 156 during assembly operations and/or during use.

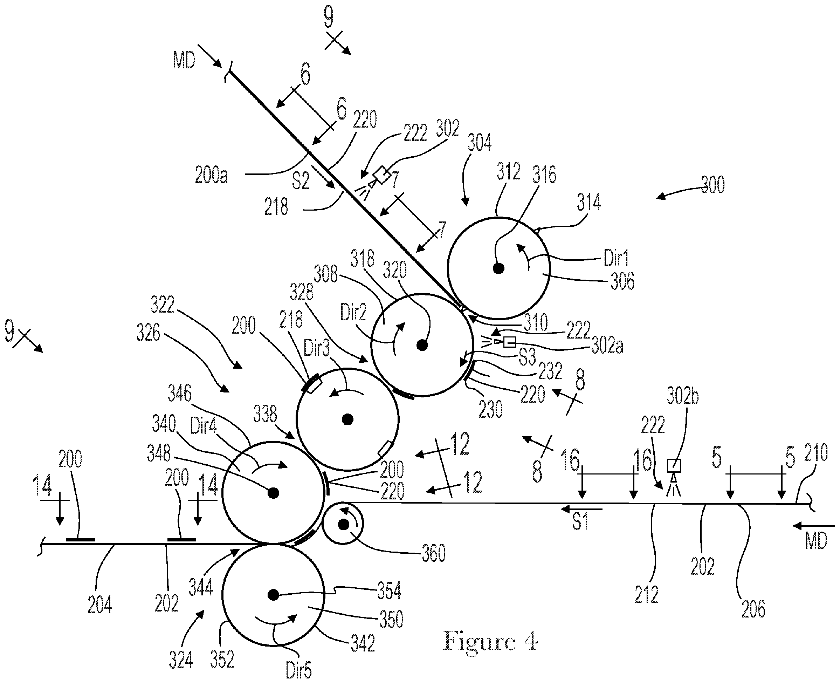

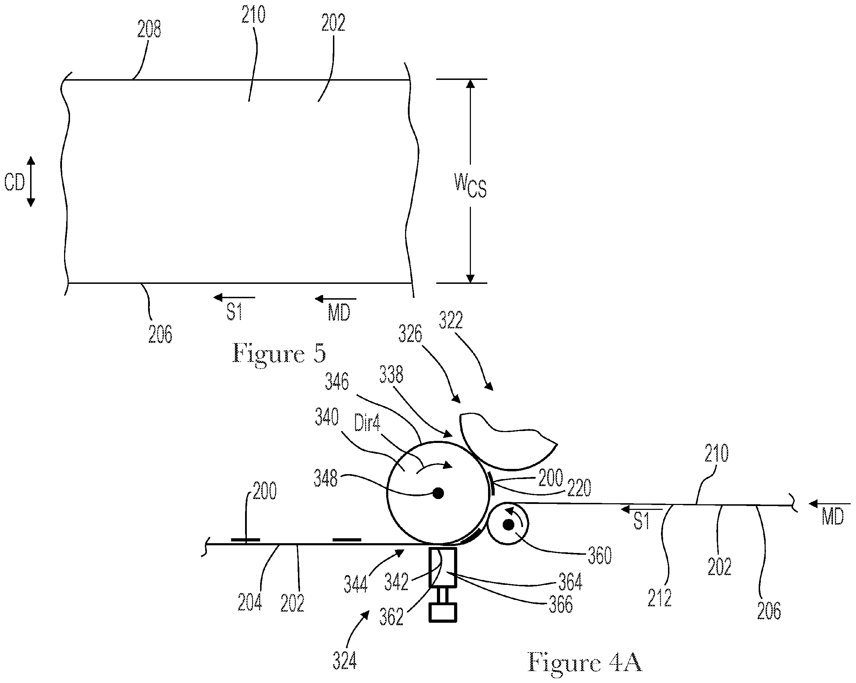

In the context of the previous discussion, various apparatuses and methods may be adapted to assemble absorbent articles 100 with first waist panels 158a and second waist panels 158b with various zones of adhesive and zones of mechanical on the first waist panel 158a and/or second waist panel 158b. For example, Figure 4 shows a schematic representation of a converting process including an apparatus or system 300 that bonds discrete elastic parts 200 under tension with an advancing carrier substrate 202 to form a laminate 204 during the assembly of an absorbent article 100. Various aspects of the apparatuses 300 and associated assemblies shown in Figure 4 are disclosed in U.S. Pub. Nos. 2020/0375807 A1; 2020/0375815 A1; 2021/0128366 A1; and 2021/0128369 A1

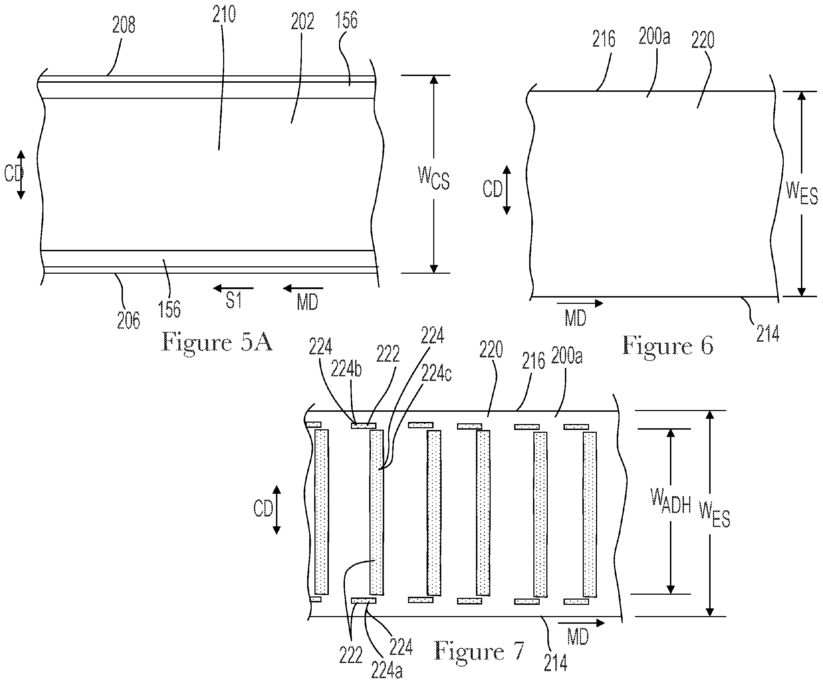

As shown in Figures 4 and 5, the carrier substrate 202 may advance in a machine direction MD at a first speed S1. The carrier substrate comprises a first longitudinal edge 206 and a second longitudinal edge 208 separated from the first longitudinal edge 206 in a cross direction CD to define a width W_{CS}. The carrier substrate 202 also includes a first surface 210 and an opposing second surface 212. As discussed in more detail below, discrete elastic parts 200 are bonded with the first surface 210 of the carrier substrate 202.

In the context of components of absorbent articles 100 discussed above and assembly processes thereof, the elastic parts 200 may be configured as waist panels 158. In some configurations, each discrete elastic part 200 may be configured as a first waist panel 158a, a second waist panel 158b, or may be a part that is subsequently cut along with the carrier substrate 202 to be formed into a first waist panel 158a and a second waist panel 158b. The carrier substrate 202 may be configured as a continuous topsheet 138, backsheet 136, or continuous laminate of a combined topsheet 138 and backsheet 136 that may also be part of a continuous length of chassis 102. The laminate 204 may be configured as a continuous length of absorbent articles 100. In some configurations, the first surface 210 of the carrier substrate 202 may correspond with the wearer facing surface 132 or the garment facing surface 134 of the topsheet 138 or backsheet 136. In some configurations, the elastic part 200 may be bonded between a topsheet 138 and a backsheet 136. For example, the elastic part 200 may be bonded with the wearer facing surface 132 of the backsheet 136, which is subsequently bonded with a topsheet 138. In another example, the elastic part 200 may be bonded with the garment facing surface 134 of the topsheet 138, which is subsequently bonded with a backsheet 136. In yet another example, the elastic part 200 may be bonded with the garment facing surface 134 of the backsheet 136, wherein the wearer facing surface 132 of the backsheet 136 may have been previously bonded with a topsheet 138 or may be subsequently bonded with a topsheet 138. In another example, the elastic part 200 may be bonded with the wearer facing surface 132 of the topsheet 136, wherein the garment facing surface 134 of the topsheet 138 may have been previously bonded with a backsheet 136 or may be subsequently bonded with a backsheet 136. As discussed above with reference to Figures 17 and 18, the waist panels 158 and chassis 102 may include various material combinations and constructions, as such, it is to be appreciated that such combinations are also applicable to the elastic substrate 200a, elastic parts 200, and the carrier substrate 202.

As shown in Figure 5A, the carrier substrate 202 may also include leg gasketing elements 156 positioned on the first surface 210 adjacent the first longitudinal edge 206 and the second longitudinal edge 208. As such, portions of the discrete elastic parts 200 may also be bonded with the leg gasketing elements 156. In some configurations, the discrete elastic parts 200 may be bonded with the carrier substrate 202 and leg gasketing elements 156 may subsequently be bonded with the carrier substrate 202. The leg gasketing elements 156 may be positioned relative the elastic part 200 such that the leg gasketing elements 156 may or may not partially cover or overlap opposing end portions of the elastic part 200. In some configurations, the leg gasketing elements 156 may be sandwiched between the elastic parts 200 and the carrier substrate 202. And in some configurations, the elastic parts 200 may be sandwiched between the leg gasketing elements 156 and the carrier substrate 202.

Referring now to Figures 4 and 6, a continuous elastic substrate 200a is advanced at a second speed S2 in a machine direction **MD,** wherein the second speed S2 is less than the first speed S1. The continuous elastic substrate 200a comprises a first longitudinal edge 214 and a second longitudinal edge 216 separated from the first longitudinal edge 214 in the cross direction CD to define a width W_{ES}. The continuous elastic substrate 200a also includes a first surface 218 and an opposing second surface 220. The continuous elastic substrate 200a is stretchable in at least one direction and is oriented such that the continuous elastic substrate 200a is stretchable in the cross direction CD. As such, the width W_{ES} of the continuous elastic substrate may be an unstretched width. In some configurations, the width W_{ES} of the continuous elastic substrate 200a may be a partially stretched width.

With continued reference to Figures 4, 6, and 7, the system 300 may include one or more adhesive applicator devices 302 that deposits adhesive 222 onto the second surface 220 of the continuous elastic substrate 200a. It is to be appreciated that the adhesive applicator device 302 may be configured in various way, such as for example, as a spray nozzle and/or a slot coating device. In some configurations, the adhesive applicator device 302 may be configured in accordance with the apparatuses and/or methods disclosed in U.S. Patent Nos. 8,186,296; 9,265,672; 9,248,054; and 9,295,590 and U.S. Pub. No. 2014/0148773 A1,

It is to be appreciated that the adhesive 222 may be applied to the continuous elastic substrate 200a to define zones 224 of adhesive 222 on the second surface 220 having various shapes and sizes relative to the continuous elastic substrate 200a. For example, as shown in Figure 7, adhesive 222 may be applied to the second surface 220 of the continuous elastic substrate 200a intermittently along the machine direction MD to define first zones 224a, second zones 224b, and third zones 224c. The first and second zones 224a, 224b may be offset from or may be coterminous with edges 214, 216, respectively, of the continuous elastic substrate 200a. In some configurations, the first and second zones 224a, 224b may be offset from, may have a shared edge with, or may overlap with the third zone 224c. In some configurations, the first and second zones 224a, 224b may extend continuously along the machine direction MD as opposed to being intermittently spaced. The third zone 224c may extend in the cross direction CD define a width W_{ADH}. In some configurations, the width W_{ADH} of adhesive 222 may be less than the width W_{ES} of the continuous elastic substrate 200a, and in some configurations, the width W_{ADH} may be equal to the width W_{ES} of the continuous elastic substrate 200a. As discussed below, the continuous elastic substrate 200a may be converted into waist panels 158 discussed herein, and as such, the first, second, and third zones 224a, 224b, 224c may correspond with the first, second, and third zones 188a, 188b, 188c, respectively, of adhesive discussed above. It is to be appreciated that adhesive 222 may be applied from one or more adhesive applicator devices 302 to define the zones 224a, 224b, 224c of adhesive 222.

As shown in Figures 4, 7, and 8, the continuous elastic substrate 200a may advance in the machine direction MD from the adhesive applicator device 302 to a cutting device 304 that cuts and separates discrete elastic parts 200 from the continuous elastic substrate 200a. As such, the discrete elastic parts 200 each include a leading edge 230 and a trailing edge 232 and defines a length L_{EP} in the machine direction MD extending from the leading edge 230 to the trailing edge 232. The elastic part 200 also includes first and second longitudinal edges 214, 216 that correspond with the longitudinal edges 214, 216 of the continuous elastic substrate 200a extending between the leading and trailing edges 230, 232. In addition, the elastic part 200 includes first and second surfaces 218, 220 that correspond with the first and second surfaces 218, 220 of the continuous elastic substrate 200a.

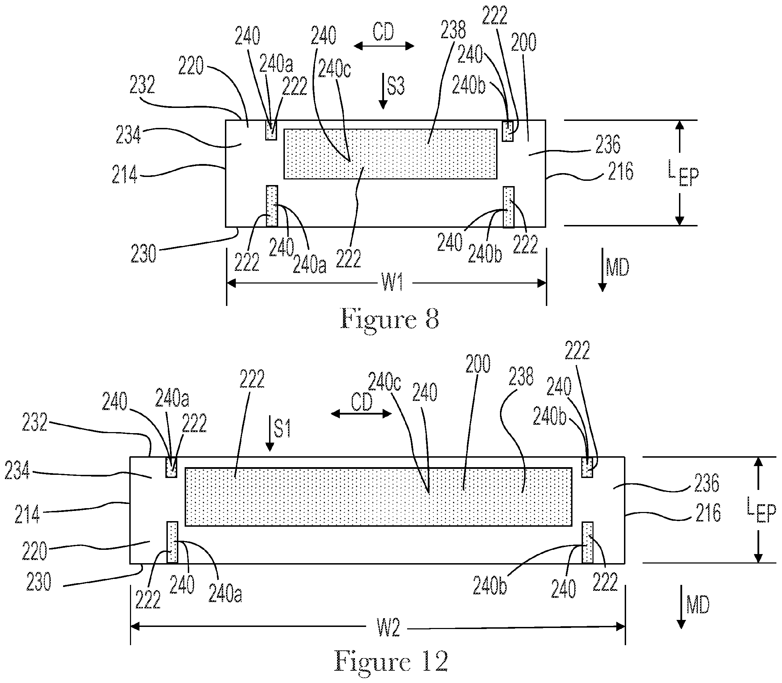

As shown in Figure 8, the discrete elastic part 200 also includes a first end region 234 adjacent the first longitudinal edge 214 and a second end region 236 adjacent the second longitudinal edge 216, wherein the second end region 236 is separated from the first end region 234 in the cross direction CD by a central region 238. As discussed above, adhesive 222 may be applied to the second surface 220 of the continuous elastic substrate 200a. As such, the discrete elastic part 200 may include zones 240 of adhesive 222 on the second surface 220. It is to be appreciated that the zones 240 of adhesive 222 may define various sizes and shapes relative to the elastic part 200. For example, as shown in Figure 8, first zones 240a of adhesive 222 may be positioned in the first end region 216, and second zones 240b of adhesive 222 may be positioned in the second end region 218. A third zone 240c of adhesive 222 may extend in the cross direction CD for less than the entire width W1 of the discrete elastic part 200. In some configurations, the third zone 240c of adhesive 222 may be positioned only on the central region 238 of the discrete elastic part 200 or may also extend into the first end region 234 and the second end region 236 of the second surface 220 of the discrete elastic part 200. As discussed below, the discrete elastic parts 200 may be converted into waist panels 158 discussed herein, and as such, the first, second, and third zones 240a, 240b, 240c may correspond with the first, second, and third zones 188a, 188b, 188c, respectively, of adhesive discussed above.

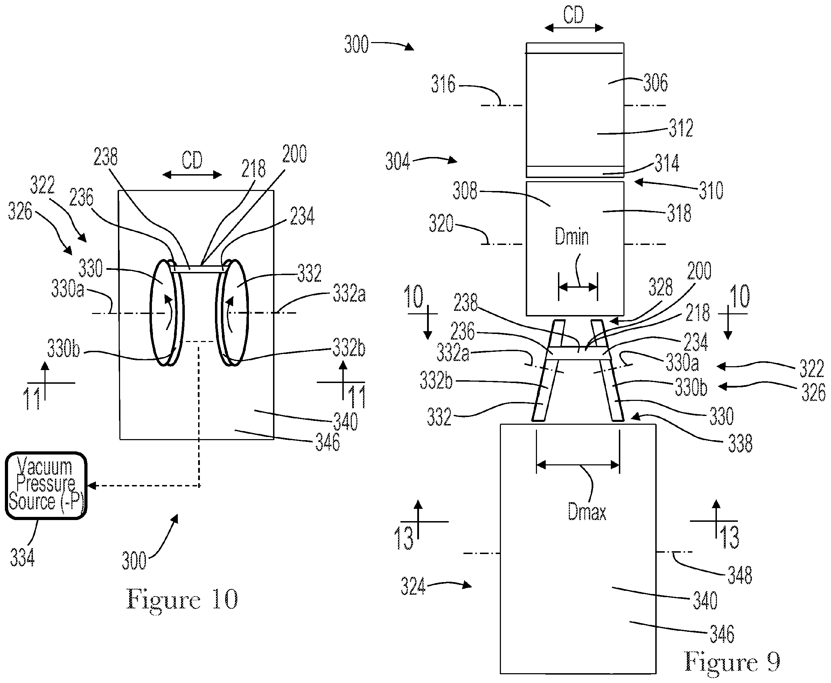

As shown in Figures 4 and 9, the cutting device 304 may include a knife roll 306 positioned adjacent an anvil roll 308 to define a nip 310 therebetween. The knife roll 306 may include an outer circumferential surface 312 and one or more blades 314 adapted to rotate about an axis 316 in a first direction Dir1. The anvil roll 308 may include an outer circumferential surface 318 adapted to rotate about an axis 320 in a second direction Dir2 opposite the first direction Dir1 such that the outer circumferential surface 318 advances at a third speed S3, wherein the third speed S3 is greater than the second speed S2. With continued reference to Figure 4, as the continuous elastic substrate 200a advances through the nip 310 between the knife roll 306 and the anvil roll 310, the blade 314 operates to cut the discrete elastic part 200 from the continuous elastic substrate 200a. Because the outer circumferential surface 318 of the anvil roll 308 advances at the third speed S3, the cut discrete elastic part 200 may then accelerate from the second speed S2 to the third speed S3 on the outer circumferential surface 318 of the anvil roll 308. It is also to be appreciated that one or more components of the cutting device 304 may be configured to operate at constant and/or variable speeds. For example, the knife roll 306 and/or the anvil roll 308 may be connected with various types of motors, such as servo motors for example, that may rotate the knife roll 306 and/or the anvil roll 308 at constant and/or variable angular velocities.

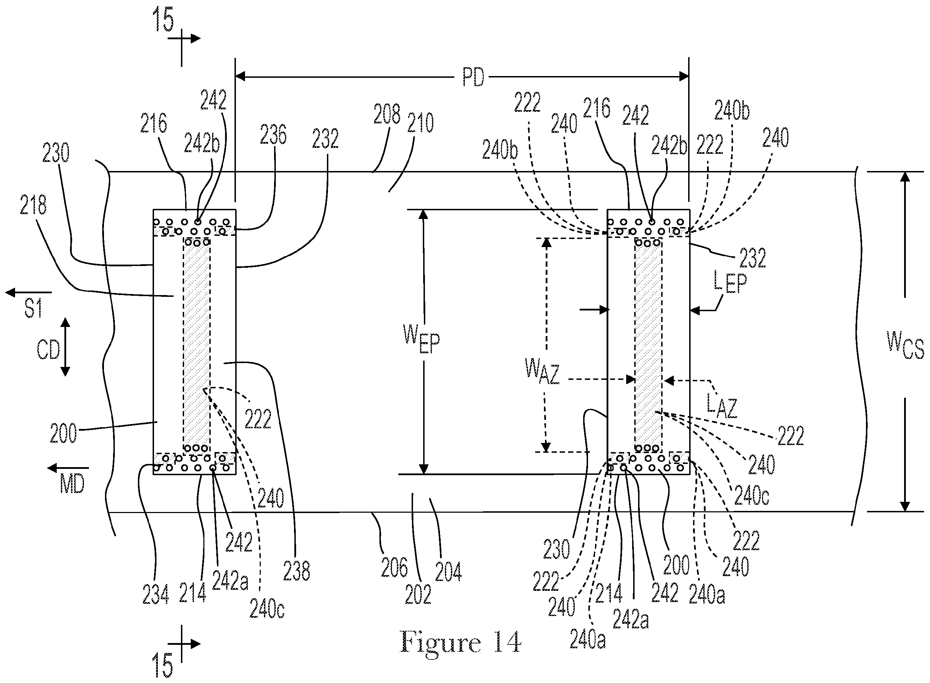

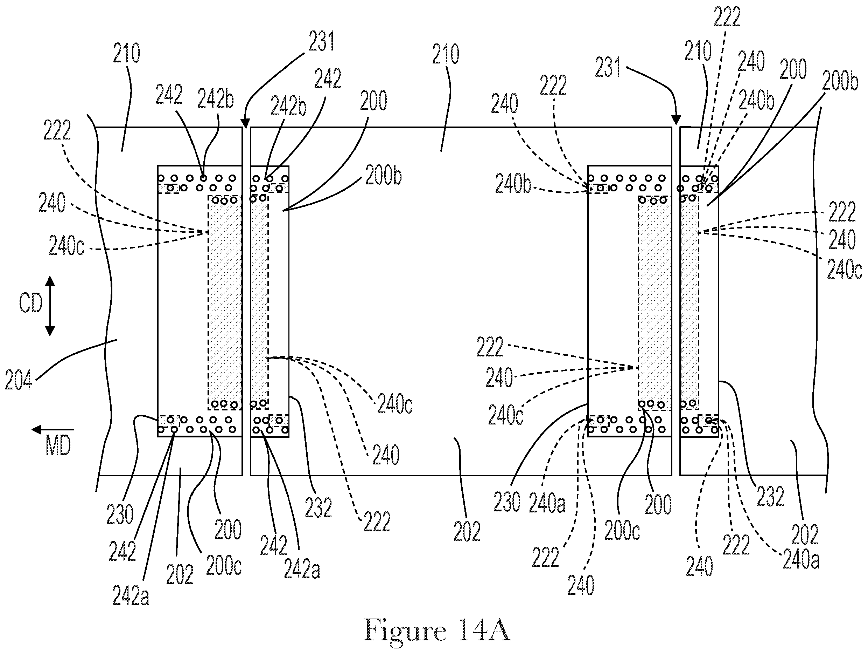

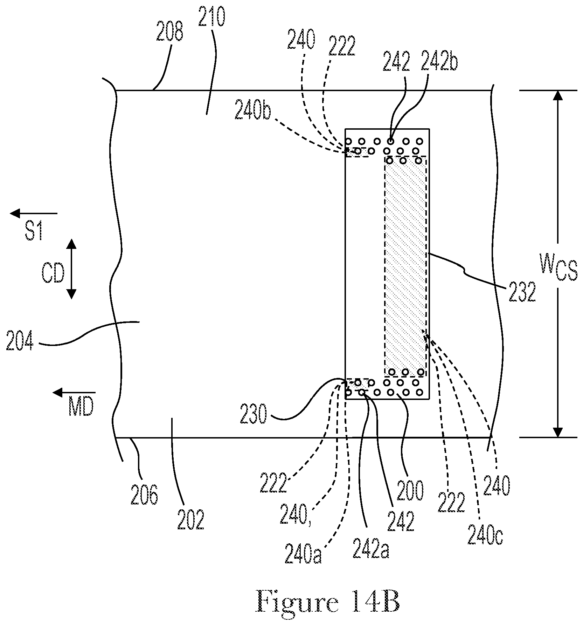

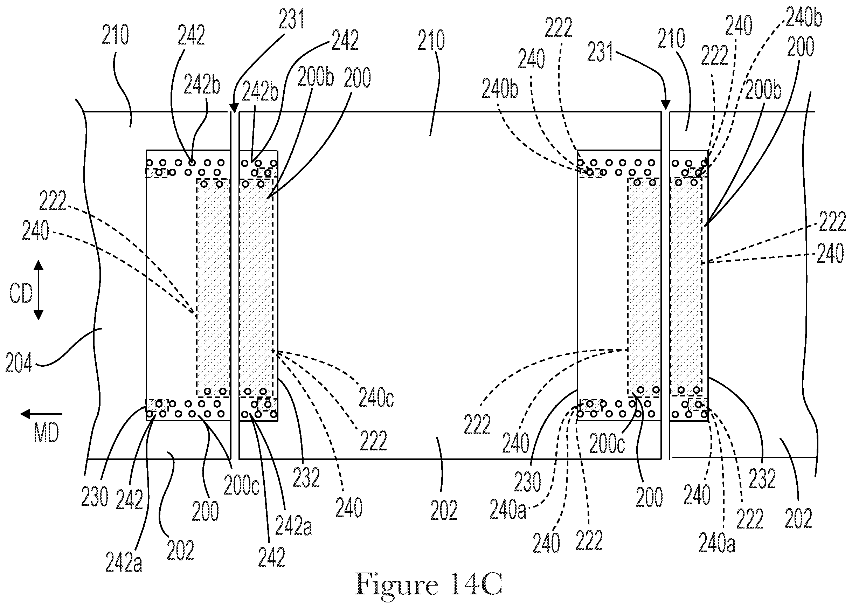

In some configurations, the third speed S3 may be equal to the first speed S1 of the advancing carrier substrate 202. In some configurations, the third speed S3 may be less than or greater than the first speed S1 of the advancing carrier substrate 202, and as such, the discrete elastic part may be accelerated or decelerated downstream of the anvil roll 308 from the third speed S3 to the first speed S1 before being combined with the carrier substrate 202. Because the first speed S1 of the carrier substrate is greater than the second speed S2, the discrete elastic parts 200 are accelerated from the second speed S2 to the first speed S1 before bonding with the carrier substrate 202. By accelerating discrete elastic parts 200 from the second speed S2 to the first speed S1, trailing edges 232 (or leading edges 230) of consecutively cut discrete elastic parts 200 may be separated from each other in the machine direction MD by a pitch distance PD, such as shown in Figure 14, which may correspond with the pitch length PL described above with reference to Figures 1A and 1B. The anvil roll 308 may also be configured to apply vacuum pressure to the discrete elastic parts 200 to help hold the discrete elastic parts 200 on the outer circumferential surface 318 as the anvil roll 308 rotates.

It is to be appreciated that the cutting device 304 may be configured in various ways. For example, in some configurations, the blade 314 may be configured such that resulting cut lines and corresponding leading edges 230 and trailing edges 232 of the discrete elastic parts 200 may be straight and/or curved. The cutting device 304 may also be adapted to cut the discrete elastic parts 200 such that material along the cut line adjacent leading edges 230 and trailing edges 232 is fused and/or pressure bonded together. It is also to be appreciated that the positions of the knife roll 306 and anvil roll 308 may be opposite to that which is illustrated in Figure 4, and as such, the discrete elastic parts 200 may remain on the outer circumferential surface 312 of the knife roll 306 as opposed to the anvil roll 308. It is also to be appreciated that the cutting device 304 may be configured to convey and/or cut the discrete elastic parts 200 in different ways.

With reference to Figure 4, the apparatus 300 may include a rotatable transfer device 322 that transfers the discrete elastic parts 200 from the cutting device 304 to a bonding device 324, which in turn, combines the elastic parts 200 with the carrier substrate 202. The transfer device 322 may also be configured to stretch the discrete elastic parts 200 in the cross direction CD. As such, the transfer device 322 may be configured as a spreader mechanism 326, such as shown in Figures 9 and 10. With continued reference to Figures 4, 9, and 10, the transfer device 322 may be positioned adjacent the anvil roll 308 to define a nip 328 therebetween. As discussed in more detail below, the discrete elastic parts 200 are received from the anvil roll 308 and the spreader mechanism 326 operates to stretch discrete elastic parts 200 in the cross direction CD. The stretched discrete elastic parts 200 are then advanced from the spreader mechanism 326 onto a rotating component of the bonding device 324, which in turn, bonds the stretched discrete elastic parts 200 onto the carrier substrate 202.

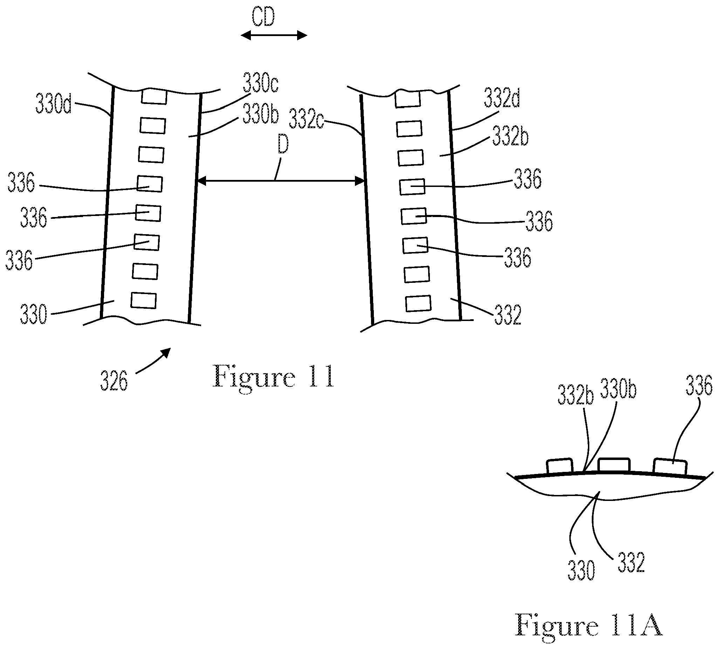

As shown in Figures 9 and 10, the spreader mechanism 326 may include a first disk 330 and a second disk 332, wherein the first disk 330 is displaced from the second disk 332 in the cross direction CD. The first disk 330 is adapted to rotate about an axis of rotation 330a and the second disk 332 is adapted to rotate about an axis of rotation 332a, wherein the first and second disks 330, 332 may rotate in a third direction Dir3 that is opposite the second direction Dir2. As shown in Figure 11, the first disk 330 includes an outer rim 330b extending axially between an inner edge 330c and an outer edge 330d, and the second disk 332 includes an outer rim 332b extending axially between an inner edge 332c and an outer edge 332d.

As shown in Figures 9-11, the first disk 330 and the second disk 332 are canted relative to each other such that the outer rims 330b, 332b are separated from each other by a distance D that increases from a minimum distance Dmin at a first location to a maximum distance Dmax at a second location. As discussed below, the discrete elastic parts 200 are transferred from the cutting device 304 onto the outer rims 330b, 332b during operation. Because the first and second disks 330, 332 are canted, rotation of the disks 330, 332 causes the rims 330b, 332b to pull on first end region 234 and the second end region 236 of discrete elastic parts 200 and stretch the central regions 238 of the discrete elastic parts 200 in the cross direction CD before the discrete elastic parts 200 are transferred to the bonding device 324. As shown in Figures 4, 8, and 12, the spreader mechanism 326 may operate to stretch the discrete elastic parts 200 in the cross direction from a first width W1 to a second width W2 that is greater than the first width W1.

With reference to Figures 4, 9, and 10, the disks 330, 332 may also be configured to help grip the opposing first and second end regions 234, 236 of the discrete elastic parts 200 during operation. For example, the first disk 330 and the second disk 332 may each be fluidly connected with a vacuum pressure source 334. As such, vacuum air pressure may be used to help hold the discrete elastic parts 200 onto the rims 330b, 332b during operation. As shown in Figures 11 and 11A, the disks 330, 332 may also include nubs 336 that protrude radially outward from the rims 330b, 332b. As such, the nubs 336 may also help prevent the first and second end regions 234, 236 of the discrete elastic parts 200 from sliding along the rims 330b, 332b while stretching the central region 238 of the discrete elastic parts 200. It is also noted that because the first and second end regions 234, 236 of the discrete elastic part 200 are held on the rims 330b, 332b during the stretching operation, the central region 238 of the discrete elastic part 200 is stretched while the first and second end regions 234, 236 may not be stretch or may be stretched to a much lesser degree than the central region 238.

As previously discussed with reference to Figure 8, the elastic part 200 may include zones 240 of adhesive 222 positioned on the central region 238 of the discrete elastic part 200 and wherein portions of the first end region 234 and the second end region 236 of the second surface 220 of the discrete elastic part 200 may not include any adhesive 222. As shown in Figures 4, 9, and 10, once transferred to the transfer device 322, the elastic parts 200 may be oriented such that the first surface 218 may be facing radially outward, and the second surface 220 and the zone 240 of adhesive 222 may be facing radially inward. As such, the arrangement of disks 330, 322 of the spreader mechanism 326 provide the ability to rotatably convey the elastic parts 200 from the cutting device 304 to the bonding device 324 with zones 240 of adhesive 222 that faces radially inward with no contact or with minimal contact of adhesive 222 with the disks 330, 332.

As discussed above, the cut discrete elastic parts 200 accelerate from the second speed S2 to the third speed S3 on the outer circumferential surface 318 of the anvil roll 308, and in some configurations, the third speed S3 may be less than or greater than the first speed S1 of the advancing carrier substrate 202. Thus, the transfer device 322 may be configured to rotate at a variable angular velocity to accelerate or decelerate the discrete elastic parts 200 to the first speed S1. For example, if the third speed S3 is less than the first speed S1, the transfer device 322 may be configured to receive the discrete elastic part 200 from the anvil roll 308 while the rims 330b, 332b of the first and second disks 330, 332 are moving through the nip 328 at the third speed S3. The angular velocity of the disks 330, 332 may then be changed to accelerate the discrete elastic part 200 to the first speed S1 before transferring the discrete elastic part 200 to the bonding device 324. In another example, if the third speed S3 is greater than the first speed S1, the angular velocity of the disks 330, 332 may be changed to decelerate the discrete elastic part 200 to the first speed S1 before transferring the discrete elastic part 200 to the bonding device 324. In situations where the third speed S3 is equal to the first speed S1, the disks 330, 332 may rotate at a constant angular velocity. It is to be appreciated that the spreader mechanism 326 may be configured in various ways to accommodate a need to rotate at variable angular velocities, such as, for example, disclosed in European Patent Publication No. EP 2260813 B1, which is incorporated by reference herein. The ability to rotate at the transfer device 326 at variable angular velocities may help reduce the need to replace components of the apparatus 300 when assembling absorbent articles 100 of smaller or larger sizes, which in turn, may require a reduction or increase in the pitch distances between consecutively cut discrete elastic parts 200.

As previously mentioned, the rotatable transfer device 322 may be configured to transfer the discrete elastic parts 200 from the cutting device 304 to a bonding device 324. As shown in Figures 4, 9, and 10, the bonding device 324 may be positioned adjacent the first and second disks 330, 332 of the spreader device 326 to define a nip 338 therebetween. In some configurations, the first and second disks 330, 332 may be configured to apply positive air pressure, sometimes referred to as blow-off air, to the discrete elastic part 200 adjacent the nip 338 to help remove the discrete elastic parts 200 from the disks 330, 332 during transfer to the bonding device 324. As discussed in more detail below, the discrete elastic parts 200 are received from the spreader mechanism 326 with the central regions 238 stretched in the cross direction CD, and the bonding device 324 transfers and bonds the discrete elastic parts 200 in the stretched state to the advancing carrier substrate 202.

It is to be appreciated that the bonding device 324 may be configured in various ways. For example, as shown in Figures 4, 9, and 10, the bonding device 324 may be configured with a pattern roll 340 and a pressing surface 342 adjacent the pattern roll 340 to define a nip 344 therebetween. The pattern roll 340 includes an outer circumferential surface 346 and rotates about an axis of rotation 348, wherein the pattern roll 340 may rotate in a fourth direction Dir4 that is opposite the third direction Dir3. In addition, pattern roll 340 may rotate such that the outer circumferential surface 346 advances at or about the first speed S1. During operation, discrete elastic parts 200 in a stretched state are transferred from the first and second disks 330, 332 to the outer circumferential surface 346 of the pattern roll 340. The pattern roll 340 rotates to advance the stretched elastic parts 200 between the outer circumferential surface 346 of the pattern roll and the advancing carrier substrate 202. In particular, the first surface 218 of the discrete elastic part 200 may be positioned in a facing relationship with and in direct contact with the outer circumferential surface 346 of the pattern roll 340. As such, the zones 240 of adhesive 222 and the second surface of the discrete elastic part 200 may be facing radially outward from the rotation axis 348. The carrier substrate 202 advances to the pattern roll 340 such that the first surface 210 of the carrier substrate 200 is in direct contact with and in a facing relationship with the outer circumferential surface 346 of the pattern roll 340. As the pattern roll 340 rotates, the second surface 220 of the discrete elastic part 200 is positioned in direct contact with and in a facing relationship with the first surface 210 of the carrier substrate 200. The combined discrete elastic part 200 and the carrier substrate 202 advance through the nip 344 between the pattern roll 340 and the pressing surface 342 to mechanically bond the discrete elastic part 200 and the carrier substrate 202 together.

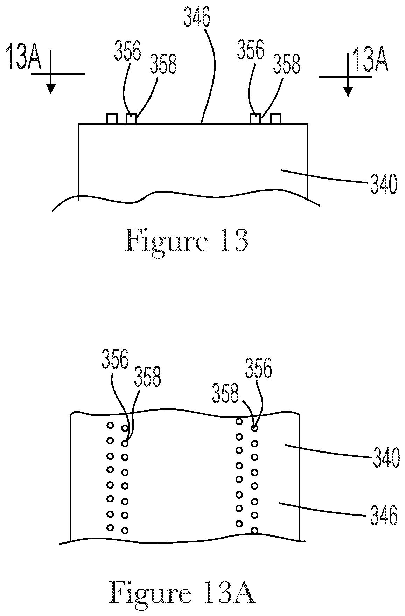

As shown in Figure 4, the bonding device 324 may be configured as a mechanical bonding device that includes an anvil roll 350. The anvil roll 350 may include an outer circumferential surface 352 and rotates about an axis of rotation 354, wherein the anvil roll 350 may rotate in a fifth direction Dir5 that is opposite the fourth direction Dir4. The outer circumferential surface 352 of the anvil roll 350 may define the pressing surface 342 operating in conjunction with the pattern roll 340. As shown in Figures 13 and 13A, the outer circumferential surface 346 of the pattern roll 340 may also comprise one or more bonding surfaces 356 defined by bonding elements 358 extending radially outward. As the pattern roll 340 rotates, the discrete elastic parts 200 and the carrier substrate 200 are advanced between the bonding surfaces 356 and the pressing surface 342 to mechanically bond or weld the elastic part 200 and the carrier substrate 202 together to create a first zone 242a of mechanical bonds 242 and a second zone 242b of mechanical bonds 242 between the elastic part 200 and the carrier substrate 202. As discussed below, the elastic parts 200 may be converted into waist panels 158 discussed herein, and as such, the first and second zones 242a, 242b may correspond with the first and second zones 190a, 190b, respectively, of mechanical bonds 190 discussed above. Heat and/or pressure between the pressing surface 342 and the pattern roll 340 may melt and bond the carrier substrate 202 and the elastic part 200 together in areas supported by the bonding surfaces 356 on the pattern roll 340. As shown in Figure 14, the mechanical bonds and/or the mechanical bonds 242 of zones 242a, 242b may have shapes that correspond with and may mirror shapes of the bonding surfaces 356.

Thus, as the laminate 204 advances through the nip 344, the carrier substrate 202 and the discrete elastic part 200 are mechanically bonded or welded together. It is to be appreciated that the bonding device 324 herein may be configured in various ways with various features described herein to bond the discrete elastic parts 200 with the carrier substrate 202. As such, the pattern roll 340 and/or anvil roll 350 may be configured to apply heat and pressure in various ways to perform mechanical bonding, such as for example, the mechanical bonding devices and methods disclosed in in U.S. Patent Nos. 4,854,984; 6,248,195; 8,778,127; 9,005,392; 9,962,297; and 10,052,237. It is also to be appreciated that the positions of the pattern roll 340 and anvil roll 350 may be opposite to that which is illustrated in Figure 4, and as such, the discrete elastic parts 200 may be transferred from the transfer device 322 to the outer circumferential surface 352 of the anvil roll 350 as opposed to the pattern roll 340. It is also to be appreciated that one or more components of the bonding device 324 may be configured to operate at constant and/or variable speeds. For example, the pattern roll 340 and/or the anvil roll 350 may be connected with various types of motors, such as servo motors for example, that may rotate the pattern roll 340 and/or the anvil roll 350 at constant and/or variable angular velocities.

In some configurations, the carrier substrate 202 may be partially wrapped around the outer circumferential surface 346 of the pattern roll 340. As such, the bonding device 324 may include one or more rolls that help guide the carrier substrate 202 to and/or from the pattern roll 340. For example, as shown in Figure 4, the bonding device may include a guide roll 360 that helps to guide the carrier substrate 202 onto the outer circumferential surface 346 of the pattern roll 340 downstream of the nip 338 where the elastic parts 202 are received from the transfer device 322 and upstream of the nip 344 between the pattern roll 340 and the pressing surface 342. The guide roll 360 may also be configured to apply pressure against the carrier substrate 202 and the elastic part 200 to help enhance the bonding of the adhesive 222 of the adhesive zone 240 and the carrier substrate 202.

It is to be appreciated that the bonding device 324 may be configured in various ways, such as with heated or unheated pattern rolls, anvil rolls and/or ultrasonic bonding devices. For example, the bonding device 324 schematically shown in Figure 4A may include the pattern roll 340 and the pressing surface 342 that comprises an energy transfer surface 362 of an ultrasonic bonding device 364. As such, the bonding device 364 may include a horn 366 and may be configured to impart ultrasonic energy to the combined elastic part 200 and the carrier substrate 202 on the pattern roll 340.

It is to be appreciated that aspects of the ultrasonic bonding device 364 may be configured in various ways, such as for example linear or rotary type configurations, and such as disclosed for example in U.S. Patent Nos. 3,113,225; 3,562,041; 3,733,238; 5,110,403; 6,036,796; 6,508,641; and 6,645,330. In some configurations, the ultrasonic bonding device 364 may be configured as a linear oscillating type sonotrode, such as for example, available from Herrmann Ultrasonic, Inc. In some configurations, the sonotrode may include a plurality of sonotrodes nested together in the cross direction CD. It is also to be appreciated that rotary horns may also be configured to rotate at constant and/or variable angular velocities.

As discussed above, the pattern roll 340 includes bonding elements 358 that extend radially outward to define bonding surfaces 356. In turn, the bonds and/or bond zones 242 between the discrete elastic part 200 and the carrier substrate 202 may have shapes that correspond with and may mirror shape of the bonding surfaces 356. It is to be appreciated that the pattern roll 340 may have various quantities and/or shapes of bonding surfaces 356 and that such bonding surfaces 356 may be positioned in various locations on the pattern roll 340. For example, as shown in Figures 13, 13A, 14, and 15, the bonding elements 358 and bonding surfaces 356 may be positioned to correspond with the first end region 234 and the second end region 236 of the discrete elastic part 200. Thus, the bonding device 340 may operate to mechanically bond the first and second end regions 234, 236 of the elastic part 200 without mechanically bonding the stretched central region 238. In some configurations, the bonding elements 358 and bonding surfaces 356 may be positioned such that mechanical bonds 242 are also applied to bond the central region 238 of the discrete elastic part 200 and the carrier substrate 202 together.

The pattern roll 340 may also be configured to apply vacuum pressure to the discrete elastic parts 200 to help hold the discrete elastic parts 200 on the outer circumferential surface 346 as the pattern roll 340 rotates. The vacuum pressure may also help hold the discrete elastic parts 200 in the stretched state while positioned on the pattern roll 340. In addition, the bonding elements 358 and bonding surfaces 356 may also help grip the elastic parts 200 and help hold the elastic parts 200 in the stretched state. In addition, the pattern roll 340 may be configured such to also apply vacuum pressure through the bonding surfaces 356 of the bonding elements 358. Further, the pattern roll 340 may be configured to interface with the first and second disks 330, 332 of the spreader mechanism 326 to help maintain the stretched state of the discrete elastic part 200 during the transfer to the pattern roll 340 at the nip 338. For example, as discussed above, the disks 330, 332 of the spreader mechanism 326 may include various quantities of nubs 336 that protrude radially outward from the rims 330b, 332b, wherein the nubs 336 may help prevent the first and second end regions 234, 236 of the elastic parts 200 from sliding toward each other along the rims 330b, 332b while stretching the discrete elastic parts 200. It is to be appreciated that the nubs 336 may be configured in various shapes and sizes, spacing, and may be constructed from various types of materials. In some configurations, the bonding elements 358 on the pattern roll 340 may be configured to intermesh with the nubs 336 protruding from the rims 330b, 332b of the first and second disks 330, 332. The intermeshing between the nubs 336 and the bonding elements 358 may help the apparatus 300 maintain the stretched state of the discrete elastic part 200 when transferring from the transfer device 322 to the bonding device 324.

As shown in Figure 4, after the discrete elastic part 200 is bonded with the carrier substrate 202 to create the laminate 204, the laminate 204 may continue to advance in the machine direction MD from the bonding device 324 and may be subjected to additional converting operations, such as cutting, folding, and/or packaging operations. In some configurations, the laminate 204 may define a continuous length of absorbent articles or may be combined with additional substrates and/or components to define a continuous length of absorbent articles. In turn, the continuous length of absorbent articles may be subjected to a final knife cut that separates discrete absorbent articles from the continuous length of absorbent articles. As previously mentioned, the discrete elastic parts 200 may correspond with waist panels 158 on the absorbent articles 100 and the carrier substrate 202 may correspond with a topsheet substrate 138 or backsheet substrate 136. In some configurations, the apparatuses and methods herein may be configured to apply discrete elastic parts 200 as discrete front and/or back waist panels 158. In some configurations, the discrete elastic parts 200 may be applied to the carrier substrate 202, and the discrete elastic parts 200 are subsequently cut during the final knife cut operation into a front waist panel 158a positioned in the front waist region 116 and a back waist panel 158b positioned in the back waist region 118, as discussed below in more detail with reference to Figures 14-14C. It is to be appreciated that such final knife cut operation may be configured to apply straight and/or curved cut lines through the carrier substrate 202 and discrete elastic parts 200. It is also to be appreciated that the carrier substrate 202 may include parts, such as laterally extending side panels for example, attached thereto upstream of the bonding device 324. As such, the system 300 may also include devices, such as rails and/or conveyors, to help guide and control the carrier substrate 202, and specifically such laterally extending features, into the bonding device 324 to help prevent unintentional bonding of such features.

As discussed above, the discrete elastic parts may be combined with the carrier substrate adhesive and/or mechanical bonds. It is to be appreciated that the adhesive and mechanical bonds may be configured in various ways. It is also to be appreciated that the zones 240 of adhesive 222 may be applied to define various different shapes and sizes with respect to the discrete elastic part 200 and/or the carrier substrate 202. For example, as shown in Figure 14, the first and second zones 240a, 240b of adhesive 222 may extend in the machine direction MD and may be conterminous with the third zone 240c and/or one of or both the leading edge 230 and the trailing edge 232 of the elastic part 200. In some configurations, the first and second zones 240a, 240b may be offset from one of or both the leading edge 230 and the trailing edge 232 of the elastic part 200. In addition, the third zone 240c of adhesive 222 may define a length L_{AZ} in the machine direction MD. In some configurations, the length L_{AZ} of the third zone 240c of adhesive 222 may extend for less than the entire length L_{EP} of the discrete elastic part 200. In some configurations, the third zone 240c of adhesive 222 may extend in the cross direction CD to be coterminous with one of or both the leading edge 230 and the trailing edge 232 of the elastic part 200. In some configurations, the length L_{AZ} of the third zone 240c of adhesive 222 may extend the entire length L_{EP} of the discrete elastic part 200 extending from the leading edge 230 to the trailing edge 232. In some configurations, the first and second zones 242a, 242b of mechanical bonds 242 may vary in width along the cross direction CD. In some configurations, the first zone 242a of mechanical bonds 242 and/or the second zone 242b of mechanical bonds 242 may be relatively wider in the cross direction adjacent the zone of adhesive 240c. In addition, the first zone 240a and/or third zone 240c of adhesive 222 may or may not overlap with the first zone 242a of mechanical bonds 242; and the second zone 240b and/or third zone 240c of adhesive 222 may or may not overlap with the second zone 242b of mechanical bonds 242.

As discussed above with reference to Figure 4, the system 300 may include an adhesive applicator device 302 that may be configured to apply adhesive 222 to the continuous elastic substrate 200a upstream of the nip 310 between the knife roll 306 and anvil roll 308. In turn, the discrete elastic parts 200 separated from the continuous elastic substrate 200a may include zones 240 of adhesive 222 that are adapted to adhesively bond the elastic part 200 with the carrier substrate 202. It is to be appreciated that the zones 240 of adhesive 222 may comprise adhesive 222 applied to the continuous elastic substrate 200a, the elastic part 200, and/or the carrier substrate 202 in various configurations and/or positions in the assembly process. For example, as shown in Figure 4, the system 300 may include an adhesive applicator device 302a that may be configured to apply adhesive 222 to the discrete elastic part 200 at a position downstream of the nip 310 between the knife roll 306 and anvil roll 308. In another example, shown in Figure 4, the apparatus 300 may include an adhesive applicator device 302b that deposits adhesive 222 onto the first surface 210 of the carrier substrate 202 to define one or more zones 240 of adhesive 222 that bonds the elastic part 200 with the carrier substrate 202. It is to be appreciated that the adhesive applicator device 302a may be configured to operate in addition to or in place of the adhesive applicators 302, 302b, and adhesive applicator device 302b may be configured to operate in addition to or in place of the adhesive applicators 302, 302a. For example, the adhesive applicator device 302 may deposit adhesive 222 onto the continuous elastic substrate 200a to define the first, second, and/or third zones 224a, 224b, 224c of adhesive 222. In another example, the adhesive applicator device 302a may deposit adhesive 222 onto the elastic part 200 to define the first, second, and/or third zones 240a, 240b, 240c of adhesive 222. In another example, the adhesive applicator device 302b may deposit adhesive 222 onto the first surface 210 of the carrier substrate 202 to define the first, second, and/or third zones 240a, 240b, 240c of adhesive 222. In another example, the adhesive applicator device 302b may deposit adhesive 222 onto the first surface 210 of the carrier substrate 202 to define the first and second zones 240a, 240b of adhesive 222, and the adhesive applicator device 302 may deposit adhesive 222 onto continuous elastic substrate 200a to define the third zone 224c of adhesive 222. In another example, the adhesive applicator device 302b may deposit adhesive 222 onto the first surface 210 of the carrier substrate 202 to define the third zone 240c of adhesive 222, and the adhesive applicator device 302 may deposit adhesive 222 onto continuous elastic substrate 200a to define the first and second zones 224a, 224b of adhesive 222. It is also to be appreciated that the adhesive applicator devices 302a, 302b may be configured in various ways, such as the adhesive applicator 302 described above, such as for example, as a spray nozzle and/or a slot coating device. It is also to be appreciated that in some configurations, the discrete elastic parts 200 may be combined with the carrier substrate 202 with only mechanical bonds and without the use of adhesive.

In accordance with the above discussion with regard to the various shapes and sizes of the zones 240 of adhesive 222, it is to be appreciated that adhesive 222 may be applied to the continuous elastic substrate 200a and/or the carrier substrate 202 in various ways to define the zones 240 of adhesive 222. For example, as discussed above with reference to Figures 4 and 7, adhesive 222 may be applied to the continuous elastic substrate 200a to define zones 224 of adhesive 222 in discrete patches separated from each other in on the continuous elastic substrate 200a in the machine direction MD. In another example, the adhesive 222 may be applied to the second surface 220 of the continuous elastic substrate 200a to extend continuously in the machine direction MD and/or the cross direction CD. In another example, shown in Figure 16, the adhesive 222 may be applied to the first surface 210 of the carrier substrate 202 in discrete zones 240 separated from each other on the carrier substrate 202 in the machine direction MD. It is to be appreciated that adhesive 222 may be applied to the continuous elastic substrate 200a, the elastic part 200, and/or the carrier substrate 202 in shapes and sizes that define the zones 240 of adhesive 222 that bond the elastic parts 200 and the carrier substrate 202 together. The discrete patches zones 240 of adhesive 222 may be separated from each other on the carrier substrate 202 in the machine direction MD by the pitch distance PD.

It is also to be appreciated that the waist panels 158 herein may be assembled in various ways, such as for example, the continuous elastic substrate and the discrete elastic parts as disclosed in U.S. Patent Nos. 6,572,595; 6,830,800; 7,087,287; and 7,803,244; and U.S. Pub. Nos. 2018/0042778 A1; 2018/0042787 A1; 2018/0042779 A1; 2018/0042780 A1; 2020/0375807 A1; 2020/0375815 A1; 2020/0375815 A1; 2021/0128366 A1; and 2021/0128369 A1,

It is to be appreciated that the continuous elastic substrate 200a and the discrete elastic parts 200 herein may be configured in various ways and may include one or more elastic materials, such as for example, elastic film and/or strands. For example, the continuous elastic substrate 200a and the discrete elastic parts 200 may be configured as a single layer of elastic film. In some configurations, the continuous elastic substrate 200a and the discrete elastic parts 200 may be configured as a laminate of two more substrates. For example, the continuous elastic substrate 200a and the discrete elastic parts 200 may be configured as an elastic film bonded in between two or more nonwoven substrates and/or may be bonded with one or more nonwoven substrates. For example, the continuous elastic substrate 200a and the discrete elastic parts 200 may be configured as a bi-laminate with an elastic film bonded with a single nonwoven substrate. In another example, the continuous elastic substrate 200a and the discrete elastic parts 200 may be configured as an elastic film bonded between two or more substrates, wherein the substrates may comprise nonwovens. It is also to be appreciated that nonwoven substrates of the elastic substrate 200a and discrete elastic parts 200 may be of the same or different material and/or basis weights. In some configurations, one more nonwoven substrates of the elastic substrate 200a and discrete elastic parts 200 may be of the same or different material and/or basis weights as one more nonwoven substrates of the carrier substrate 202.

It is also to be appreciated that the continuous elastic substrate 200a and the discrete elastic parts 200 may be assembled in various ways, such as for example, as disclosed in U.S. Patent Nos. 6,572,595; 6,830,800; 7,087,287; and 7,803,244; and U.S. Pub. Nos. 2018/0042778 A1; 2018/0042787 A1; 2018/0042779 A1; and 2018/0042780 A1,

As previously mentioned, apparatuses and methods to assemble the waist panels and/or bond the waist panels with other absorbent article components may be adapted to assemble absorbent articles 100 with first waist panels 158a and second waist panels 158b, such as discussed above. Figure 14A is a view of the laminate 204 of Figure 14 showing elastic parts 200 and the carrier substrate 202 after being subjected to a final knife cut operation that applies cut lines 231 through the carrier substrate 202 and discrete elastic parts 200. As such, the discrete elastic parts 200 may be cut into a first elastic part 200b and a second elastic part 200c. In some configurations, the first elastic part 200b may correspond with a front waist panel 158a positioned in the front waist region 116, and the second elastic part 200c may correspond with a back waist panel 158b positioned in the back waist region 118. In some configurations, a curved cut line 231 may be adapted to create an umbilical cord notch, such as disclosed in U.S. Patent No. 8,608,720 and U.S. Pub. No. 2017/0246052 A1,

In another example configuration of Figure 14B, the zones 240 adhesive 222 may extend to or close to the trailing edge 232 of the elastic part. As mentioned above with some configurations, the elastic parts 200 may not be cut and may rather correspond with a first waist panel 158a or second waist panel 158b. For example, the elastic part 202 shown in Figure 14B may correspond with a first waist panel 158a or a second waist panel 158b. It is to be appreciated that such a configuration shown in Figure 14B could also be subject to a final knife cut. For example, Figure 14C is a view of the laminate 204 showing elastic parts 200 and the carrier substrate 202 after being subjected to a final knife cut operation that applies cut lines 231 through the carrier substrate 202 and discrete elastic parts 200. As such, the discrete elastic parts 200 may be cut into a first elastic part 200b and a second elastic part 200c. In some configurations, the first elastic part 200b may correspond with a front waist panel 158a positioned in the front waist region 116, and the second elastic part 200c may correspond with a back waist panel 158b positioned in the back waist region 118. In some configurations, a curved cut line 231 may be adapted to create an umbilical cord notch, such as disclosed in U.S. Patent No. 8,608,720 and U.S. Publication No. 2017/0246052 A1,

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article comprising:
a front waist region (116), a back waist region (118), and a crotch region (119) disposed between the front and back waist regions (116, 118);
a chassis (102) comprising a topsheet (138), a backsheet (136), and an absorbent core (140) positioned between the topsheet and the backsheet;
a first leg gasketing element (156) and a second leg gasketing element (156) connected with the chassis (102) and extending from the front waist region (116) to the back waist region (118);
a first waist panel (158a) positioned in the front waist region (116) or the back waist region (118), the first waist panel (158a) comprising an inboard lateral edge (172), an outboard lateral edge (170), a first longitudinal edge (180), and a second longitudinal edge (182), wherein the first waist panel (158a) further comprises a first lateral end region (184) adjacent the first longitudinal edge (180) and a second lateral end region (186) adjacent the second longitudinal edge (182), wherein the first and second lateral end regions (184, 186) are separated by a central region (178);
a first zone (190a) of mechanical bonds (190) in the first lateral end region (184), the first zone (190a) of mechanical bonds (190) bonding the first waist panel (158a) with at least one of the chassis (102) and the first leg gasketing element (156);
a second zone (190b) of mechanical bonds (190) in the second lateral end region (186), the second zone (190b) of mechanical bonds (190) bonding the first waist panel (158a) with at least one of the chassis (102) and the second leg gasketing element (156);
a first zone (188a) of adhesive (188) in the first lateral end region (184) adjacent the inboard lateral edge (172) and bonding the first waist panel (158a) with at least one of the chassis (102) and the first leg gasketing element (156);
a second zone (188b) of adhesive (188) in the second lateral end region (186) adjacent the inboard lateral edge (172) and bonding the first waist panel (156a) with at least one of the chassis (102) and the second leg gasketing element (156); and
wherein the first waist panel (158a) is unattached to the chassis (102) between the first and second lateral end regions (184, 186).

2. The absorbent article of claim 1, further comprising:
a third zone (188c) of adhesive (188) adj acent the outboard lateral edge (170) and extending laterally through the central region (178), the third zone (188c) of adhesive (188) connecting the first waist panel (158a) with the chassis (102); and
wherein the first waist panel (158a) is unattached to the chassis (102) between the first and second lateral end regions (184, 186) and between the inboard lateral edge (172) and the third zone (188c) of adhesive (188).

3. The absorbent article of claim 2, wherein a portion of the first zone (190a) of mechanical bonds (190) and a portion of the second zone (190b) of mechanical bonds (190) overlap with portions of the third zone (188c) of adhesive (188).

4. The absorbent article of any of the preceding claims, wherein a portion of the first zone (190a) of mechanical bonds (190) overlaps with a portion of the first zone (188a) of adhesive (188).

5. The absorbent article of any of the preceding claims, wherein the first zone (190a) of mechanical bonds (190) comprises a first lateral width and the first zone (188a) of adhesive (188) comprises a second lateral width, wherein the first lateral width is greater than the second lateral width.

6. The absorbent article of any of the preceding claims, wherein the first zone (190a) of mechanical bonds (190) comprises:
a first lateral width adjacent the outboard lateral edge (170) of the first waist panel (158a);
a second lateral width adjacent the inboard lateral edge (172) of the first waist panel (158a); and
wherein the first lateral width is greater than the second lateral width.

7. The absorbent article of any of the preceding claims, wherein the first zone (188a) of adhesive (188) extends longitudinally from the inboard lateral edge (172) to the outboard lateral edge (170) of the first waist panel (158a).

8. The absorbent article of any of the preceding claims, wherein the first waist panel (158a) comprises a longitudinal length defined by a distance extending between the inboard lateral edge (172) and the outboard lateral edge (170), and wherein the first zone (188a) of adhesive (188) extends longitudinally for a distance that is less than half of the longitudinal length of the first waist panel (158a).

9. The absorbent article of claim 8, wherein the first zone (188a) of adhesive (188) extends longitudinally from the inboard lateral edge (172).

10. The absorbent article of any of the preceding claims, wherein the first zone (188a) of adhesive (188) extends longitudinally offset from the inboard lateral edge (172).

11. The absorbent article of any of the preceding claims, further comprising a first waist edge (120), a second waist edge (122) longitudinally separated from the first waist edge (120), and wherein the outboard lateral edge (170) of the first waist panel (158a) is coterminous with the first waist edge (120).

12. The absorbent article of any of the preceding claims, further comprising a second waist panel (158b) positioned in the front waist region (116); and wherein the first waist panel (158a) is positioned in the rear waist region (118).

13. The absorbent article of any of the preceding claims, wherein the first waist panel (158a) comprises an elastic material.

14. The absorbent article of claim 13, wherein the first waist panel (158a) comprises an elastic film bonded with a nonwoven in a stretched state.

15. The absorbent article of any of the preceding claims, wherein at least one of the first zone (190a) of mechanical bonds (190) and the second zone (190b) of mechanical bonds (190) extends between the outboard lateral edge (170) and the inboard lateral edge (172).

## Patentansprüche

1. Absorptionsartikel, umfassend:
einen vorderseitigen Taillenbereich (116), einen rückseitigen Taillenbereich (118) und einen Schrittbereich (119), der zwischen dem vorderseitigen und dem rückseitigen Taillenbereich (116, 118) eingerichtet ist;
eine Grundeinheit (102), umfassend eine Oberschicht (138), eine Unterschicht (136) und einen zwischen der Oberschicht und der Unterschicht angeordneten Absorptionskern (140);
ein erstes Beindichtungselement (156) und ein zweites Beindichtungselement (156), die mit der Grundeinheit (102) verknüpft sind und sich von dem vorderseitigen Taillenbereich (116) zu dem rückseitigen Taillenbereich (118) erstrecken;
ein erstes Taillenfeld (158a), das in dem vorderseitigen Taillenbereich (116) oder dem rückseitigen Taillenbereich (118) angeordnet ist, das erste Taillenfeld (158a) umfassend einen innenliegenden Seitenrand (172), einen außenliegenden Seitenrand (170), einen ersten Längsrand (180) und einen zweiten Längsrand (182), wobei das erste Taillenfeld (158a) ferner einen ersten seitlichen Endbereich (184) benachbart zu dem ersten Längsrand (180) und einen zweiten seitlichen Endbereich (186) benachbart zu dem zweiten Längsrand (182) umfasst, wobei der erste und der zweite seitliche Endbereich (184, 186) durch einen Zentralbereich (178) getrennt sind;
einen ersten Bereich (190a) aus mechanischen Bindungen (190) in dem ersten seitlichen Endbereich (184), wobei der erste Bereich (190a) aus mechanischen Bindungen (190) das erste Taillenfeld (158a) mit wenigstens einem von der Grundeinheit (102) und dem ersten Beindichtungselement (156) verbindet;
einen zweiten Bereich (190b) aus mechanischen Bindungen (190) in dem zweiten seitlichen Endbereich (186), wobei der zweite Bereich (190b) aus mechanischen Bindungen (190) das erste Taillenfeld (158a) mit wenigstens einem von der Grundeinheit (102) und dem zweiten Beindichtungselement (156) verbindet;
einen ersten Bereich (188a) aus Klebstoff (188) in dem ersten seitlichen Endbereich (184), der benachbart zu dem innenliegenden Seitenrand (172) ist und der das erste Taillenfeld (158a) mit wenigstens einem von der Grundeinheit (102) und dem ersten Beindichtungselement (156) verbindet;
einen zweiten Bereich (188b) aus Klebstoff (188) in dem zweiten seitlichen Endbereich (186), der benachbart zu dem innenliegenden Seitenrand (172) ist und der das erste Taillenfeld (156a) mit wenigstens einem von der Grundeinheit (102) und dem zweiten Beindichtungselement (156) verbindet; und
wobei das erste Taillenfeld (158a) zwischen dem ersten und dem zweiten seitlichen Endbereich (184, 186) nicht an der Grundeinheit (102) befestigt ist.

2. Absorptionsartikel nach Anspruch 1, ferner umfassend:
einen dritten Bereich (188c) aus Klebstoff (188), der benachbart zu dem außenliegenden Seitenrand (170) ist und sich seitlich durch den Zentralbereich (178) erstreckt, wobei der dritte Bereich (188c) aus Klebstoff (188) das erste Taillenfeld (158a) mit der Grundeinheit (102) verknüpft; und
wobei das erste Taillenfeld (158a) zwischen dem ersten und dem zweiten seitlichen Endbereich (184, 186) und zwischen dem innenliegenden Seitenrand (172) und dem dritten Bereich (188c) aus Klebstoff (188) nicht an der Grundeinheit (102) befestigt ist.

3. Absorptionsartikel nach Anspruch 2, wobei ein Abschnitt des ersten Bereichs (190a) aus mechanischen Bindungen (190) und ein Abschnitt des zweiten Bereichs (190b) aus mechanischen Bindungen (190) mit Abschnitten des dritten Bereichs (188c) aus Klebstoff (188) überlappen.

4. Absorptionsartikel nach einem der vorstehenden Ansprüche, wobei ein Abschnitt des ersten Bereichs (190a) aus mechanischen Bindungen (190) mit einem Abschnitt des ersten Bereichs (188a) aus Klebstoff (188) überlappt.

5. Absorptionsartikel nach einem der vorstehenden Ansprüche, wobei der erste Bereich (190a) aus mechanischen Bindungen (190) eine erste seitliche Breite umfasst und der erste Bereich (188a) aus Klebstoff (188) eine zweite seitliche Breite umfasst, wobei die erste seitliche Breite größer als die zweite seitliche Breite ist.

6. Absorptionsartikel nach einem der vorstehenden Ansprüche, wobei der erste Bereich (190a) aus mechanischen Bindungen (190) umfasst:
eine erste seitliche Breite benachbart zu dem außenliegenden Seitenrand (170) des ersten Taillenfelds (158a);
eine zweite seitliche Breite benachbart zu dem innenliegenden Seitenrand (172) des ersten Taillenfelds (158a); und
wobei die erste seitliche Breite größer als die zweite seitliche Breite ist.

7. Absorptionsartikel nach einem der vorstehenden Ansprüche, wobei sich der erste Bereich (188a) aus Klebstoff (188) längs von dem innenliegenden Seitenrand (172) zu dem außenliegenden Seitenrand (170) des ersten Taillenfelds (158a) erstreckt.

8. Absorptionsartikel nach einem der vorstehenden Ansprüche, wobei das erste Taillenfeld (158a) eine Längslänge umfasst, die durch einen Abstand definiert ist, der sich zwischen dem innenliegenden Seitenrand (172) und dem außenliegenden Seitenrand (170) erstreckt, und wobei sich der erste Bereich (188a) aus Klebstoff (188) längs über einen Abstand, der weniger als die Hälfte der Längslänge des ersten Taillenfelds (158a) beträgt, erstreckt.

9. Absorptionsartikel nach Anspruch 8, wobei sich der erste Bereich (188a) aus Klebstoff (188) längs von dem innenliegenden Seitenrand (172) erstreckt.

10. Absorptionsartikel nach einem der vorstehenden Ansprüche, wobei sich der erste Bereich (188a) aus Klebstoff (188) längs versetzt von dem innenliegenden Seitenrand (172) erstreckt.

11. Absorptionsartikel nach einem der vorstehenden Ansprüche, ferner umfassend einen ersten Taillenrand (120), einen zweiten Taillenrand (122), der längs von dem ersten Taillenrand getrennt ist, und wobei der außenliegende Seitenrand (170) des ersten Taillenfelds (158a) an den ersten Taillenrand (120) angrenzt.

12. Absorptionsartikel nach einem der vorstehenden Ansprüche, ferner umfassend ein zweites Taillenfeld (158b), das in dem vorderseitigen Taillenbereich (116) angeordnet ist; und wobei das erste Taillenfeld (158a) in dem rückseitigen Taillenbereich (118) angeordnet ist.

13. Absorptionsartikel nach einem der vorstehenden Ansprüche, wobei das erste Taillenfeld (158a) ein elastisches Material umfasst.

14. Absorptionsartikel nach Anspruch 13, wobei das erste Taillenfeld (158a) eine elastische Folie umfasst, die mit einem Vlies in einem gedehnten Zustand verbunden ist.

15. Absorptionsartikel nach einem der vorstehenden Ansprüche, wobei sich wenigstens einer des ersten Bereichs (190a) aus mechanischen Bindungen (190) und des zweiten Bereichs (190b) aus mechanischen Bindungen (190) zwischen dem außenliegenden Seitenrand (170) und dem innenliegenden Seitenrand (172) erstreckt.

## Revendications

1. Article absorbant comprenant :
une région de taille avant (116), une région de taille arrière (118), et une région d'entrejambe (119) disposée entre les régions de taille avant et arrière (116, 118) ;
un châssis (102) comprenant une feuille de dessus (138), une feuille de fond (136), et une âme absorbante (140) positionnée entre la feuille de dessus et la feuille de fond ;
un premier élément faisant jointure de jambe (156) et un second élément faisant jointure de jambe (156) relié au châssis (102) et s'étendant de la région de taille avant (116) à la région de taille arrière (118) ;
un premier pan de taille (158a) positionné dans la région de taille avant (116) ou la région de taille arrière (118), le premier pan de taille (158a) comprenant un bord latéral intérieur (172), un bord latéral extérieur (170), un premier bord longitudinal (180) et un second bord longitudinal (182), dans lequel le premier pan de taille (158a) comprend en outre une première région extrême latérale (184) adjacente au premier bord longitudinal (180) et une seconde région extrême latérale (186) adjacente au second bord longitudinal (182), dans lequel les première et seconde régions extrêmes latérales (184, 186) sont séparées par une région centrale (178) ;
une première zone (190a) de liaisons mécaniques (190) dans la première région extrême latérale (184), la première zone (190a) de liaisons mécaniques (190) liant le premier pan de taille (158a) à au moins l'un parmi le châssis (102) et le premier élément faisant jointure de jambe (156) ;
une seconde zone (190b) de liaisons mécaniques (190) dans la seconde région extrême latérale (186), la seconde zone (190b) de liaisons mécaniques (190) liant le premier pan de taille (158a) à au moins l'un parmi le châssis (102) et le second élément faisant jointure de jambe (156) ;
une première zone (188a) d'adhésif (188) dans la première région extrême latérale (184) adjacente au bord latéral intérieur (172) et liant le premier pan de taille (158a) à au moins l'un parmi le châssis (102) et le premier élément faisant jointure de jambe (156) ;
une deuxième zone (188b) d'adhésif (188) dans la seconde région extrême latérale (186) adjacente au bord latéral intérieur (172) et liant le premier pan de taille (156a) à au moins l'un parmi le châssis (102) et le second élément faisant jointure de jambe (156) ; et
dans lequel le premier pan de taille (158a) n'est pas fixé au châssis (102) entre les première et seconde régions extrêmes latérales (184, 186).

2. Article absorbant selon la revendication 1, comprenant en outre :
une troisième zone (188c) d'adhésif (188) adjacente au bord latéral extérieur (170) et s'étendant latéralement à travers la région centrale (178), la troisième zone (188c) d'adhésif (188) reliant le premier pan de taille (158a) au châssis (102) ; et
dans lequel le premier pan de taille (158a) n'est pas fixé au châssis (102) entre les première et seconde régions extrêmes latérales (184, 186) et entre le bord latéral intérieur (172) et la troisième zone (188c) d'adhésif (188).

3. Article absorbant selon la revendication 2, dans lequel une partie de la première zone (190a) de liaisons mécaniques (190) et une partie de la seconde zone (190b) de liaisons mécaniques (190) chevauchent des parties de la troisième zone (188c) d'adhésif (188).

4. Article absorbant selon l'une quelconque des revendications précédentes, dans lequel une partie de la première zone (190a) de liaisons mécaniques (190) chevauche une partie de la première zone (188a) d'adhésif (188).

5. Article absorbant selon l'une quelconque des revendications précédentes, dans lequel la première zone (190a) de liaisons mécaniques (190) comprend une première largeur latérale et la première zone (188a) d'adhésif (188) comprend une seconde largeur latérale, dans lequel la première largeur latérale est supérieure à la seconde largeur latérale.

6. Article absorbant selon l'une quelconque des revendications précédentes, dans lequel la première zone (190a) de liaisons mécaniques (190) comprend :
une première largeur latérale adjacente au bord latéral extérieur (170) du premier pan de taille (158a) ;
une seconde largeur latérale adjacente au bord latéral intérieur (172) du premier pan de taille (158a) ; et
dans lequel la première largeur latérale est supérieure à la seconde largeur latérale.

7. Article absorbant selon l'une quelconque des revendications précédentes, dans lequel la première zone (188a) d'adhésif (188) s'étend longitudinalement du bord latéral intérieur (172) au bord latéral extérieur (170) du premier pan de taille (158a).

8. Article absorbant selon l'une quelconque des revendications précédentes, dans lequel le premier pan de taille (158a) comprend une longueur longitudinale définie par une distance s'étendant entre le bord latéral intérieur (172) et le bord latéral extérieur (170), et dans lequel la première zone (188a) d'adhésif (188) s'étend longitudinalement sur une distance qui est inférieure à la moitié de la longueur longitudinale du premier pan de taille (158a).

9. Article absorbant selon la revendication 8, dans lequel la première zone (188a) d'adhésif (188) s'étend longitudinalement à partir du bord latéral intérieur (172).

10. Article absorbant selon l'une quelconque des revendications précédentes, dans lequel la première zone (188a) d'adhésif (188) s'étend longitudinalement décalée du bord latéral intérieur (172).

11. Article absorbant selon l'une quelconque des revendications précédentes, comprenant en outre un premier bord de taille (120), un second bord de taille (122) longitudinalement séparé du premier bord de taille (120), et dans lequel le bord latéral extérieur (170) du premier pan de taille (158a) coïncide avec le premier bord de taille (120).

12. Article absorbant selon l'une quelconque des revendications précédentes, comprenant en outre un second pan de taille (158b) positionné dans la région de taille avant (116) ; et dans lequel le premier pan de taille (158a) est positionné dans la région de taille arrière (118).

13. Article absorbant selon l'une quelconque des revendications précédentes, dans lequel le premier pan de taille (158a) comprend un matériau élastique.

14. Article absorbant selon la revendication 13, dans lequel le premier pan de taille (158a) comprend un film élastique lié à un non-tissé dans un état étiré.

15. Article absorbant selon l'une quelconque des revendications précédentes, dans lequel au moins l'une parmi la première zone (190a)de liaisons mécaniques (190) et la seconde zone (190b) de liaisons mécaniques (190) s'étend entre le bord latéral extérieur (170) et le bord latéral intérieur (172).
